(19) Europäisches Patentamt
European Patent Office
Office européen des brevets

(11) EP 1 179 336 B1

(12) # FASCICULE DE BREVET EUROPEEN

(45) Date de publication et mention de la délivrance du brevet:
**10.09.2008 Bulletin 2008/37**

(51) Int Cl.:
*A61K 8/39* (2006.01) *A61K 8/86* (2006.01)
*A61K 8/34* (2006.01) *A61Q 5/10* (2006.01)

(21) Numéro de dépôt: **01402086.1**

(22) Date de dépôt: **01.08.2001**

(54) **Composition pour la teinture d'oxydation des fibres kératiniques comprenant un polymère amphiphile cationique, un alcool gras oxyalkyléné ou glycérolé et un solvant hydroxylé**

Zusammensetzung für die oxidative Färbung von keratinischen Fasern, die ein kationisches amphiphilisches Polymer, eine alkoxylierte oder glyzerinierte Fettsäure, und ein Hydroxyllösungsmittel enhält

Oxidative dyeing composition for keratinic fibres comprising an cationic amphiphylic polymer, an oxyalkylated or glycerolated fatty alcohol, and a hydroxyl solvent

(84) Etats contractants désignés:
**AT BE CH CY DE DK ES FI FR GB GR IE IT LI LU MC NL PT SE TR**

(30) Priorité: **11.08.2000 FR 0010593**

(43) Date de publication de la demande:
**13.02.2002 Bulletin 2002/07**

(73) Titulaire: **L'ORÉAL**
**75008 Paris (FR)**

(72) Inventeurs:
- **Laurent, Florence**
**92500 Asnieres (FR)**
- **Allard, Delphine**
**92700 Colombes (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 673 641** **EP-A- 0 943 320**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 179 336 B1

Printed by Jouve, 75001 PARIS (FR)

## Description

La présente invention concerne une composition pour la teinture d'oxydation des fibres kératiniques, en particulier des fibres kératiniques humaines telles que les cheveux, comprenant, dans un milieu approprié pour la teinture, au moins un colorant d'oxydation, au moins un polymère amphiphile cationique comportant au moins une chaîne grasse, et une association comprenant : (I) au moins un alcool gras oxyalkyléné ou glycérolé et (II) au moins un solvant hydroxylé de poids moléculaire inférieur à 250, dans une proportion telle que le ratio pondéral (I) / (II) est supérieur à 1.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para-phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Il est important de pouvoir bien localiser le produit de coloration d'oxydation à l'application sur les cheveux afin d'une part qu'il ne coule pas sur le visage ou en dehors des zones que l'on se propose de teindre, et d'obtenir d'autre part une coloration uniforme et régulière sur l'ensemble de la chevelure.
Il est également important que les compositions contenant les colorants d'oxydation avant mélange avec un agent oxydant soient stables dans le temps en particulier au niveau rhéologique.

Dans la demande de brevet européen N°- 0943320, pour formuler des produits de teinture d'oxydation satisfaisant à ces données, on a préconisé d'utiliser des polymères associatifs cationiques choisis parmi les celluloses et les hydroxyéthylcelluloses quaternisées comportant au moins une chaîne grasse.
Outre les bonnes qualités qu'offrent lesdites compositions à l'application, elles engendrent des colorations puissantes et chromatiques (lumineuses) avec de faibles sélectivités et de bonnes ténacités vis à vis des agents chimiques (shampooing, permanentes) ou naturels (lumière, transpiration) tout en apportant aux cheveux de bonnes propriétés cosmétiques.

Néanmoins, la demanderesse a constaté que la couleur du mélange extemporané obtenu à partir de la composition contenant les colorants d'oxydation et de l'oxydant évolue trop rapidement; cette couleur fonce très vite pendant le temps de pause dudit mélange sur les cheveux, ce qui indique une oxydation prématurée des colorants pouvant perturber le résultat tinctorial, et constitue également un problème esthétique.

Voici maintenant qu'après d'importantes recherches menées sur la question, la Demanderesse vient de découvrir qu'il est possible d'obtenir une composition pour la teinture d'oxydation dont le mélange avec l'oxydant évolue beaucoup moins rapidement en couleur et permet donc de réduire l'oxydation prématurée des colorants d'oxydation, si on introduit dans la composition une association définie ci-après.

Cette découverte est à la base de la présente invention.

La présente invention a ainsi pour objet une composition destinée à la teinture d'oxydation des fibres kératiniques, en particulier des fibres kératiniques humaines, telles que les cheveux, comprenant, dans un milieu approprié pour la teinture, au moins un colorant d'oxydation, au moins un polymère amphiphile cationique comportant au moins une chaîne grasse et caractérisée par le fait qu'elle comprend en outre une association comprenant :

(I) au moins un alcool gras oxyalkyléné ou glycérolé et,
(II) au moins un solvant hydroxylé de poids moléculaire inférieur à 250,

dans une proportion telle que le ratio pondéral (I)/(II) est supérieur à 1.

Un autre objet de l'invention porte sur une composition prête à l'emploi pour la teinture d'oxydation des fibres kératiniques, en particulier des fibres kératiniques humaines, telles que les cheveux, comprenant, dans un milieu approprié pour la teinture, au moins un colorant d'oxydation, au moins un polymère amphiphile cationique comportant au moins une chaîne grasse, au moins un agent oxydant et une association selon l'invention telle que décrite ci-avant.

Par composition prête à l'emploi, on entend au sens de l'invention, toute composition destinée à être appliquée immédiatement sur les fibres kératiniques; elle peut donc être stockée telle quelle avant utilisation ou résulter du mélange extemporané de deux ou plusieurs compositions.

L'invention vise également un procédé de teinture des fibres kératiniques, et en particulier des fibres kératiniques humaines telles que les cheveux, consistant à appliquer sur les fibres une composition (A) contenant, dans un milieu

approprié pour la teinture, au moins un colorant d'oxydation, au moins un polymère amphiphile cationique comportant au moins une chaîne grasse et une association telle que définie ci-avant, la couleur étant révélée à pH alcalin, neutre ou acide à l'aide d'une composition (B) contenant au moins un agent oxydant qui est mélangée juste au moment de l'emploi à la composition (A) ou qui est appliquée séquentiellement sans rinçage intermédiaire.

L'invention a également pour objet un dispositif de teinture ou " kit " à plusieurs compartiments pour la teinture d'oxydation des fibres kératiniques, en particulier des fibres kératiniques humaines telles que les cheveux, dont le premier compartiment contient au moins un colorant d'oxydation, au moins un polymère amphiphile cationique comportant au moins une chaîne grasse et une association telle que décrite ci-avant, et le second compartiment au moins un agent oxydant.

Mais d'autres caractéristiques, aspects, objets et avantages de l'invention apparaîtront encore plus clairement à la lecture de la description et des exemples qui suivent.

Polymères amphiphiles cationiques

La structure chimique des polymères amphiphiles est caractérisée par la présence de zones hydrophiles assurant la solubilité dans l'eau, et de zones hydrophobes par lesquelles les polymères, dans un milieu aqueux, s'assemblent entre eux ou avec les parties hydrophobes d'autres molécules. On les qualifie également de "polymères associatifs" c'est à dire des polymères hydrosolubles capables, dans un milieu aqueux, de s'*associer* réversiblement entre eux ou avec d'autres molécules.

Les polymères amphiphiles cationiques comportant au moins une chaîne grasse utilisés dans la présente invention peuvent notamment être choisis parmi les dérivés de cellulose quaternisée, les polyuréthanes cationiques, les polyvinyllactames cationiques et le terpolymère acrylique dont la constitution est donnée ci-après.

Les dérivés de cellulose quaternisée sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA.

Les polyuréthanes cationiques

La famille de polyuréthanes amphiphiles cationiques selon l'invention a été décrite par la demanderesse dans la demande de brevet français N°-0009609; elle peut être représentée par la formule générale (Ia) suivante :

$$R\text{-}X\text{-}(P)_n\text{-}[L\text{-}(Y)_m]_r\text{-}L'\text{-}(P')_p\text{-}X'\text{-}R' \qquad (Ia)$$

dans laquelle :

R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe.

Dans un mode de réalisation préféré des polyuréthanes de la présente invention, les seuls groupements

hydrophobes sont les groupes R et R' aux extrémités de chaîne.

Une famille préférée de polyuréthanes amphiphiles cationiques est celle correspondant à la formule (Ia) décrite ci-dessus et dans laquelle :

R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes amphiphiles cationiques est celle correspondant à la formule (Ia) ci-dessus dans laquelle :

R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes amphiphiles cationiques est celle correspondant à la formule (Ia) ci-dessus dans laquelle :

R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes amphiphiles cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes :

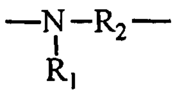
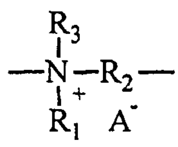
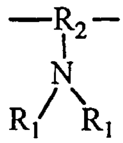
ou
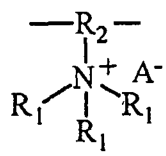
pour X

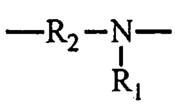
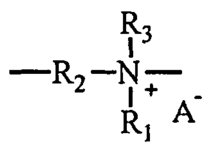
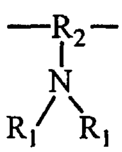
ou
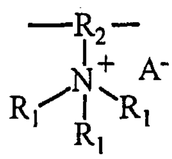
pour X'

dans lesquelles :

$R_2$ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
$R_1$ et $R_3$, identiques ou différents, désignent un radical alkyle ou alcényle en $C_1$-$C_{30}$, linéaire ou ramifié, un radical aryle, l'un au moins des atomes de carbone pouvant être remplacé par un hétéroatome choisi parmi N, S, O, P ;
A est un contre-ion physiologiquement acceptable.

Les groupements L, L' et L" représentent un groupe de formule :

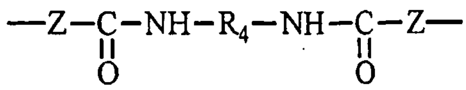

dans laquelle :

Z représente -O-, -S- ou -NH- ; et
$R_4$ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes :

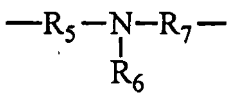

ou

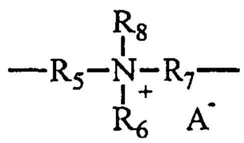

ou

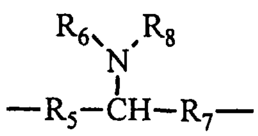

ou

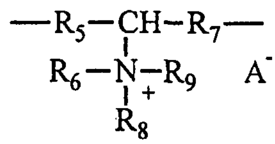

ou

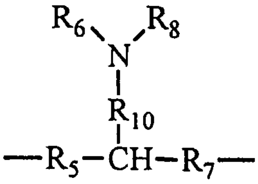

ou

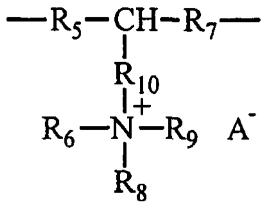

dans lesquelles :

$R_5$ et $R_7$ ont les mêmes significations que $R_2$ défini précédemment;

$R_6$, $R_8$ et $R_9$ ont les mêmes significations que $R_1$ et $R_3$ définis précédemment ;

$R_{10}$ représente un groupe alkylène, linéaire ou ramifié, éventuellement insaturé et pouvant contenir un ou plusieurs hétéroatomes choisis parmi N, O, S et P, et $A^-$ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes amphiphiles cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (Ia) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine. Ce type de composés peut être représenté par l'une des formules suivantes :

$$HZ\text{-}(P)_n\text{-}ZH,$$

ou

$$HZ\text{-}(P')_p\text{-}ZH$$

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (Ia) est un diisocyanate correspondant à la formule :

$$O{=}C{=}N\text{-}R_4\text{-}N{=}C{=}O$$

dans laquelle $R_4$ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (Ia) est un composé hydrophobe destiné à former les groupes hydrophobes terminaux du polymère de formule (Ia).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné $\alpha$-hydroxyle.

Le groupe hydrophobe du polyuréthane de formule (I) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane amphiphile cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (Ia) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes amphiphiles cationiques comportant un tel groupe.

Lesdits polyuréthanes amphiphiles cationiques sont hydrosolubles ou hydrodispersibles.

<u>Les polyvinyllactames cationiques</u>

Les polymères poly(vinyllactame) cationiques selon l'invention comprennent :

- -a) au moins un monomère de type vinyl lactame ou alkylvinyllactame;
- -b) au moins un monomère de structures (Ib) ou (IIb) suivantes :

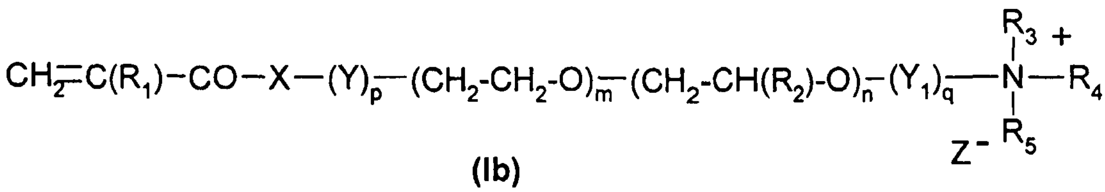

**(Ib)**

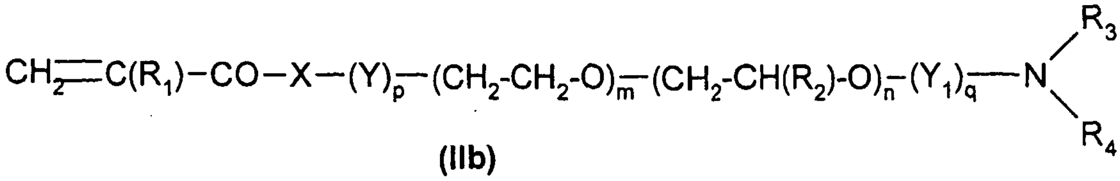

**(IIb)**

dans lesquelles :

X désigne un atome d'oxygène ou un radical $NR_6$,

$R_1$ et $R_6$ désignent, indépendamment l'un de l'autre, un atome d'hydrogène ou un radical alkyl linéaire ou ramifié en $C_1$-$C_5$,
$R_2$ désigne un radical alkyle linéaire ou ramifié en $C_1$-$C_4$,
$R_3$, $R_4$ et $R_5$ désignent, indépendamment l'un de l'autre, un atome d'hydrogène, un radical alkyle linéaire ou ramifié en $C_1$-$C_{30}$ ou un radical de formule (IIIb) :

$$-(Y_2)_r-(CH_2-CH(R_7)-O)_x-R_8 \quad \textbf{(IIIb)}$$

Y , $Y_1$ et $Y_2$ désignent, indépendamment l'un de l'autre, un radical alkylène linéaire ou ramifié en $C_2$-$C_{16}$,
$R_7$ désigne un atome d'hydrogène, ou un radical alkyle linéaire ou ramifié en $C_1$-$C_4$ ou un radical hydroxyalkyle linéaire ou ramifié en $C_1$-$C_4$,
$R_8$ désigne un atome d'hydrogène ou un radical alkyle linéaire ou ramifié en $C_1$-$C_{30}$,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,

sous réserve que :

- l'un au moins des substituants $R_3$, $R_4$, $R_5$ ou $R_8$ désigne un radical alkyle linéaire ou ramifié en $C_9$-$C_{30}$,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.
De préférence le contre ion $Z^-$ des monomères de formule (Ib) est choisi parmi les ions halogénures, les ions phosphates, l'ion méthosulfate, l'ion tosylate.
De préférence $R_3$, $R_4$ et $R_5$ désignent, indépendamment l'un de l'autre, un atome d'hydrogène ou un radical alkyle linéaire ou ramifié en $C_1$-$C_{30}$.
Plus préférentiellement, le monomère b) est un monomère de formule (Ib) pour laquelle, encore plus préférentiellement, m et n sont égaux à zéro.
Le monomère vinyl lactame ou alkylvinyllactame est de préférence un composé de structure (IVb) :

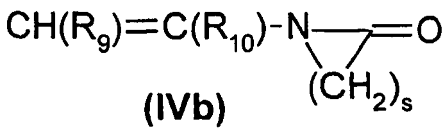

**(IVb)**

dans laquelle :

s désigne un nombre entier allant de 3 à 6,
$R_9$ désigne un atome d'hydrogène ou un radical alkyle en $C_1$-$C_5$,
$R_{10}$ désigne un atome d'hydrogène ou un radical alkyle en $C_1$-$C_5$,
sous réserve que l'un au moins des radicaux $R_9$ et $R_{10}$ désigne un atome d'hydrogène.

Encore plus préférentiellement, le monomère (IVb) est la vinylpyrrolidone.
Les polymères poly(vinyllactame) cationiques selon l'invention peuvent également contenir un ou plusieurs monomères supplémentaires, de préférence cationiques ou non ioniques.
A titre de composés plus particulièrement préférés selon l'invention, on peut citer les terpolymères suivants comprenant au moins :

a)-un monomère de formule (IVb),
b)-un monomère de formule (Ib) dans laquelle p=1, q=0, $R_3$ et $R_4$ désignent, indépendamment l'un de l'autre, un atome d'hydrogène ou un radical alkyle en $C_1$-$C_5$ et $R_5$ désigne un radical alkyle en $C_9$-$C_{24}$ et
c)-un monomère de formule (IIb) dans laquelle $R_3$ et $R_4$ désignent, indépendamment l'un de l'autre, un atome d'hydrogène ou un radical alkyle en $C_1$-$C_5$.

Encore plus préférentiellement, on utilisera les terpolymères comprenant, en poids, 40 à 95% de monomère (a), 0,1 à 55% de monomère (c) et 0,25 à 50% de monomère (b). De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone /diméthylaminopropylméthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les terpolymères acryliques décrits ci-après

Parmi les polymères amphiphiles cationiques selon l'invention on peut aussi citer les terpolymères acryliques tels que décrits dans la demande de brevet EP- 1090 623 et qui sont constitués de :

- de 5 à 80% en poids, préférentiellement de 15 à 70% en poids et plus préférentiellement de 40 à 70% en poids, d'un monomère acrylate (a), choisi parmi un acrylate d'alkyle en $C_1$-$C_6$ et un méthacrylate d'alkyle en $C_1$-$C_6$;
- de 5 à 80% en poids, préférentiellement de 10 à 70% en poids et plus préférentiellement de 20 à 60% en poids, d'un monomère (b), choisi parmi un composé vinylique héterocyclique contenant au moins un atome d'azote ou de soufre, un (méth)acrylamide, un (méth)acrylate de mono- ou di-($C_1$-$C_4$)alkylamino($C_1$-$C_4$)alkyle et un mono ou di-($C_1$-$C_4$)alkylamino($C_1$-$C_4$)alkyl (méth)acrylamide;
- de 0,1 à 30% en poids, préférentiellement de 0,1 à 10% en poids d'un monomère (c), choisi parmi (i)un uréthane produit par réaction entre un isocyanate insaturé monoéthylénique et un tensioactif non ionique à terminaison $C_1$-$C_4$ alkoxy; (ii) un copolymère à blocs de 1,2-butylène oxyde et de 1,2-éthylène oxyde; (iii) un monomère tensioactif insaturé éthylénique copolymérisable obtenu par condensation d'un tensioactif non-ionique avec un acide carboxylique insaturé $\alpha$,$\beta$-éthylénique ou son anhydride; (iv) un monomère tensioactif choisi parmi les produits de réaction de type urée d'un monoisocyanate insaturé monoéthylénique avec un tensioactif non-ionique présentant une fonction amine; (v)un éther de (méth)allyle de formule $CH_2$= $CR_1CH_2OA_mB_nA_pR_2$ dans lequel $R_1$ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur à 100, m et p désignent zéro ou un nombre entier inférieur à n et $R_2$ est un groupe hydrophobe d'au moins 8 atomes de carbone et préférentiellement en $C_8$-$C_{30}$; et (vi)un monomère non-ionique de type uréthane produit par réaction d'un tensioactif non ionique monohydrique avec un isocyanate insaturé monoéthylénique; les pourcentages en poids de monomères étant basés sur le poids total des monomères constituant le terpolymère.

Des monomères acrylates (a) préférés comprennent notamment les acrylates d'alkyle en $C_2$-$C_6$. L'acrylate d'éthyle est tout particulièrement préféré.

Comme exemples de monomères (b) préférés, il faut citer le méthacrylate de N,N-diméthylaminoéthyle (DMAEMA), acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé $\alpha$,$\beta$-éthylénique ou son anhydride, de préférence les acides mono ou di-carboxyliques en $C_3$-$C_4$ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en $C_{10}$-$C_{30}$ alkoxylés avec 2 à 100, et de préférence de 5 à 50 moles d'oxyde d'alkylène, comme par exemple les éthers de polyéthylène glycol et d'alcools gras en $C_{10}$-$C_{30}$ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion. Des terpolymères conformes à l'invention et leurs méthodes de préparation sont notamment décrits dans les

demandes EP-A-0824914 et EP-A-0825200.
Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en $C_{10}$-$C_{30}$ polyoxyéthyléné à 20 moles d'oxyde d'éthylène sous forme de dispersion aqueuse à 20% de Matière Active. En plus de ces monomères, les terpolymères peuvent contenir d'autres monomères qui permettent de réticuler lesdits terpolymères. Ces monomères sont utilisés dans des proportions assez faibles, jusqu'à 2% en poids par rapport au poids total des monomères utilisés pour préparer les terpolymères. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes.
Des monomères de réticulation peuvent notamment être des divinyl-benzènes, des trivinyl-benzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octa-allyl saccharoses, des cycloparaffines, des cyclooléfines et du N-méthylène-bis-acrylamide.
Dans la présente invention, les polymères amphiphiles cationiques comportant au moins une chaîne grasse sont utilisés de préférence en une quantité pouvant varier d'environ 0,01 à 3% en poids par rapport au poids total de la composition de teinture. Plus préférentiellement, cette quantité varie d'environ 0,02 à 0,5% en poids.

Alcool gras oxyalkyléné ou glycérolé

Par alcool gras oxyalkyléné, on entend tout alcool gras pur de structure suivante :

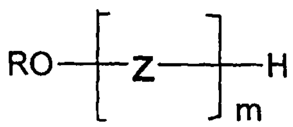

dans laquelle,

R désigne un radical saturé ou insaturé, linéaire ou ramifié, comportant de 8 à 40 atomes de carbone et de préférence de 8 à 30,
Z représente un radical oxyéthyléné (i) et/ou oxypropyléné $(ii)_1$ et $(ii)_2$ de formules respectives suivantes :

$-CH_2-CH_2-O-$ (i)

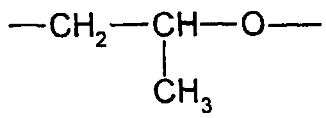
$(ii)_1$

$-CH_2-CH_2-CH_2-O-_2$ (ii)

m représente le nombre de groupements oxyde d'éthylène (i) et/ou oxyde de propylène $(ii)_1$ ou $(ii)_2$ , allant de 1 et 250 et de préférence de 2 à 100.

Par alcool gras glycérolé, on entend tout alcool gras pur de structure suivante :

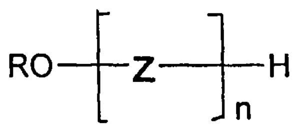

dans laquelle,

R désigne un radical saturé ou insaturé, linéaire ou ramifié, comportant de 8 à 40 atomes de carbone et de préférence de 8 à 30,

Z représente un radical glycérolé (iii) de formule suivante :

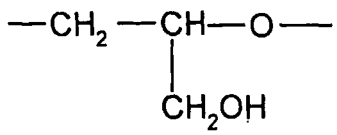

(iii)

n représente le nombre de groupements glycérol (iii) et est compris entre 1 et 30 et de préférence entre 1 et 10.

La composition selon l'invention peut renfermer des mélanges de ces alcools gras oxyalkylénés ou glycérolés.

Des alcools gras oxyalkylénés particulièrement préférés selon l'invention sont des alcools gras saturés ou non, linéaires ou ramifiés, comportant de 10 à 20 atomes de carbone et 2 à 40 groupements oxyde d'éthylène.

Comme composés de type alcool gras oxyalkyléné, on peut notamment citer les produits commercialisés suivants :

Mergital LM2 (COGNIS) [alcool laurique 2 OE ];
Ifralan L12 (IFRACHEM) et Rewopal 12 (GOLDSCHMITDT) [alcool laurique 12 OE];
Empilan KA 2.5/90FL (ALBRIGHT & WILSON) et Mergital BL309 (COGNIS) [alcool décylique 3 OE];
Empilan KA 5/90FL (ALBRIGHT & WILSON) et Mergital BL589 (COGNIS) [alcool décylique 5 OE];
Brij 58 (UNIQUEMA) et Simulsol 58 (SEPPIC) [alcool cétylique 20 OE];
Eumulgin 05 (COGNIS) [alcool oleocétylique 5 OE];
Mergital OC30 (COGNIS) [alcool oleocétylique 30 OE];
Brij 72 (UNIQUEMA) [alcool stéarylique 2 OE];
Brij 76 (UNIQUEMA) [alcool stéarylique 10 OE];
Brij 78P (UNIQUEMA) [alcool stéarylique 20 OE];
Brij 700 (UNIQUEMA) [alcool stéarylique 100 OE];
Eumulgin B1 (COGNIS) [alcool cétylstéarylique 12 OE];
Eumulgin L (COGNIS) [alcool cétylique 9 OE et 2 OP];
Witconol APM (GOLDSCHMIDT) [alcool myristique 3 OP].

Comme composés de type alcool gras glycérolé, on peut notamment citer l'alcool laurique à 4 moles de glycérol (nom INCI : POLYGLYCERYL-4 LAURYL ETHER), l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol , l'alcool oléocétylique à 6 moles de glycérol, et l'octadécanol à 6 moles de glycérol.

L'alcool gras peut représenter un mélange d'alcools gras, ce qui signifie que dans un produit commercial peuvent coexister plusieurs espèces d'alcools gras oxyalkylénés ou glycérolés sous forme d'un mélange.

Les alcools gras oxyalkylénés ou glycérolés représentent 0,05 à 50%, et de préférence 2 à 40% en poids du poids total de la composition.

Solvant hydroxylé

Par solvant hydroxylé, on désigne au sens de l'invention, un composé linéaire, ramifié ou cyclique, saturé ou insaturé, portant au moins une fonction -OH sur la chaîne.
De préférence, il s'agit d'un composé ayant de 2 à 12 atomes de carbone, et encore plus préférentiellement de 2 à 8. Avantageusement, ce composé comporte 2 ou 3 atomes de carbone.

Les solvants hydroxylés utilisés selon invention peuvent être choisis notamment parmi les alcools éthylique, propylique, n-butylique, les polyols en $C_2$-$C_6$ dont le 2-méthyl-1,3-butanediol, le 2,2-diméthyl-1,3-propanediol, le 2-méthyl-1,3-propanediol, le 2-méthyl-2,4-pentanediol, le 1,5-pentanediol, le 3-méthyl-1,5-pentanediol, et plus particulièrement la glycérine, le propylène glycol, le dipropylèneglycol, et le 1,3-propanediol.
Les éthers de polyols peuvent également être utilisés selon l'invention à condition de posséder au moins une fonction hydroxyle libre; ils peuvent être choisis notamment parmi les éthers en $C_1$-$C_8$ de glycol en $C_2$-$C_9$.
Parmi les éthers en $C_1$-$C_8$ de glycol en $C_2$-$C_9$, on peut citer notamment :

(i) les éthers en $C_1$-$C_8$ de glycol en $C_2$ et plus particulièrement les alkyléthers en $C_4$-$C_8$ de glycol en $C_2$ tels que le monobutyléther d'éthylèneglycol, ainsi que les aryléthers en $C_6$-$C_8$ de glycol en $C_2$ tels que le monophényléther d'éthylèneglycol ou le monobenzyléther d'éthylèneglycol,

(ii) les éthers en $C_1$-$C_8$ de glycol en $C_3$-$C_9$ dont plus particulièrement (a) les alkyléthers en $C_1$-$C_8$ de glycol en $C_3$-$C_9$ tels que le monométhyléther de propylèneglycol, le monoéthyléther de propylèneglycol, le monométhyléther et le monoéthyléther du diéthylèneglycol, le monométhyléther de dipropylèneglycol, le monométhyléther de tripropylèneglycol, ainsi que (b) les aryléthers en $C_6$-$C_8$ de glycol en $C_3$-$C_9$ tels que le monophényléther de propylèneglycol, le monobenzyléther de propylèneglycol, le monophényléther de diéthylèneglycol et le monobenzyléther de diéthylèneglycol.

Les solvants hydroxylés de poids moléculaire inférieur à 250 sont présents dans la composition conforme à l'invention dans des proportions généralement comprises entre 0,01 et 25% en poids, et encore plus particulièrement de 0,1 à 20% en poids par rapport au poids total de la composition.

Dans la composition conforme à l'invention le ratio pondéral entre le ou les alcools gras oxyalkylénés ou glycérolés et le ou les solvants hydroxylés est supérieur à 1, de préférence compris entre 1 et 30 et encore plus préférentiellement entre 1,5 et 20.

Colorants d'oxydation

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide .

On peut notamment citer,:

- (I) les paraphénylènediamines de formule (I) suivante et leurs sels d'addition avec un acide :

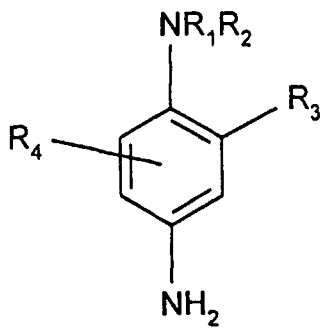

(I)

dans laquelle :

**$R_1$** représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;

**$R_2$** représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

**$R_1$** et **$R_2$** peuvent également former avec l'atome d'azote qui les porte un hétérocycle azoté éventuellement substitué;

**$R_3$** représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,

**$R_4$** représente un atome d'hydrogène, d'halogène ou un radical alkyle en $C_1$-$C_4$.

Parmi les groupements azotés de la formule (I) ci-dessus, on peut citer notamment les radicaux amino, monoalkyl ($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

- **(II)** Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide :

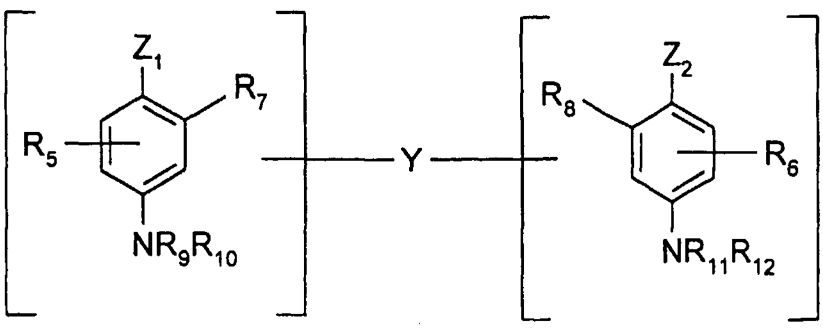

**(II)**

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ;

  étant entendu que les composés de formule (II) ne comportent qu'un seul bras de liaison Y par molécule.

Parmi les groupements azotés de la formule (II) ci-dessus, on peut citer notamment les radicaux amino, monoalkyl ($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane

ou l'un de leurs sels d'addition avec un acide sont particulièrement préférés.

- **(III)** les para-aminophénols répondant à la formule (III) suivante, et leurs sels d'addition avec un acide :

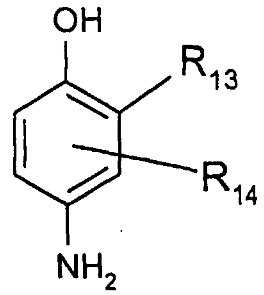

**(III)**

dans laquelle :

**$R_{13}$** représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

**$R_{14}$** représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl-pyrazolo-[1,5-a]-pyrimidine-3,7-diamine, la 2,6-diméthyl-pyrazolo-[1,5-a]-pyrimidine-3,7-diamine, la 2, 5, N7, N7-tetraméthyl-pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 4,5-diamino-1-hydroxyéthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino 1-méthyl-4-méthylamino-pyrazole, le 3,5-diamino-4-(β-hydroxyéthyl)amino 1-méthyl-pyrazole, et leurs sels d'addition avec un acide.

Selon la présente invention, les bases d'oxydation représentent de préférence de 0,0005 à 12% en poids environ du poids total de la composition.

Les coupleurs utilisables dans le procédé de teinture selon l'invention sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, le chlorure de 3-(4-hydroxy-1-méthyl-1H-indol-5-yle-méthyl)-1-méthyl-pyridinium, la 6-hydroxy-indoline, la 2,6-dihydroxy-4-méthyl-pyridine, le 1-H-3-méthyl-pyrazole-5-one, le 1-phényl-3-méthyl-pyrazole-5-one, et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Dans la composition prête à l'emploi selon l'invention, la composition colorante (A) et/ou la composition oxydante (B) peuvent en outre plus particulièrement contenir, au moins un polymère cationique différent de ceux de la présente invention, ou un polymère amphotère.

Au sens de la présente invention, l'expression "polymère cationique" désigne tout polymère contenant des groupements cationiques et/ou des groupements ionisables en groupements cationiques.

Lesdits polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant

au moins un des motifs de formules (I), (II), (III) ou (IV) suivantes:

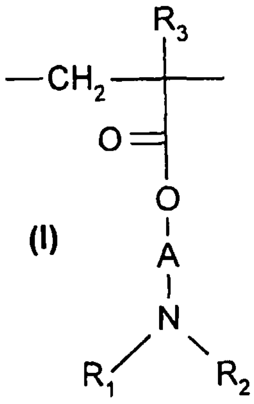
(I)

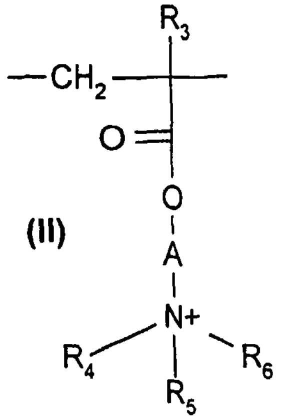
(II)

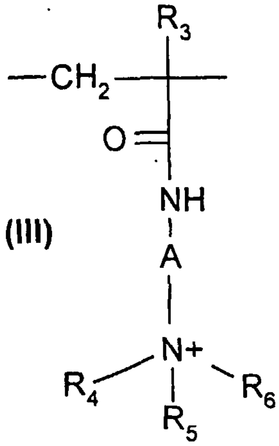
(III)

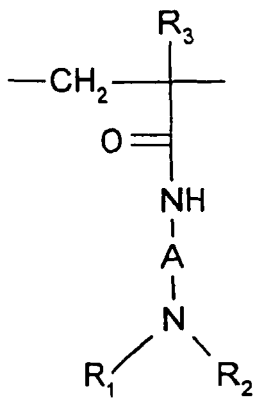
(IV)

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyltriméthyl-ammonium décrits par exemple

dans la demande de brevet EP-A-080976 et vendus sous la dénomination BINA QUAT P 100 par la société CIBA GEIGY,

- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP .

**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .

**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "Celquat L 200" et "Celquat H 100" par la Société National Starch.

**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium. De tels produits sont commercialisés notamment sous les dénominations commerciales de JAGUAR C13 S, JAGUAR C 15, JAGUAR C 17 ou JAGUAR C162 par la société MEYHALL.

**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

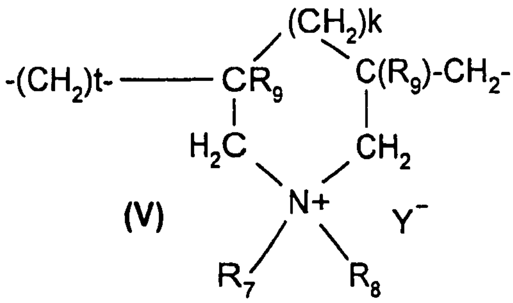

**(V)**

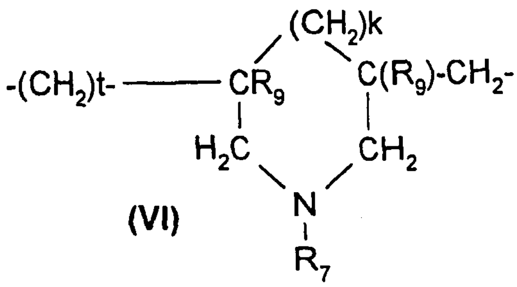

**(VI)**

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

**(10)** Le polymère de diammonium quaternaire contenant des motifs récurrents répondant à la formule :

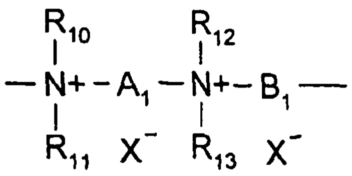

(VII)

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien
$R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
$X^-$ désigne un anion dérivé d'un acide minéral ou organique;
A1, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - $(CH_2)n$-CO-D-OC-$(CH_2)n$- dans lequel D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- .

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule (VIII) suivante:

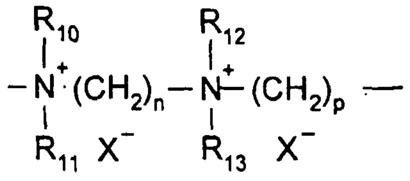

(VIII)

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

**(11)** Les polymères de polyammonium quaternaire constitués de motifs récurrents de formule (IX) :

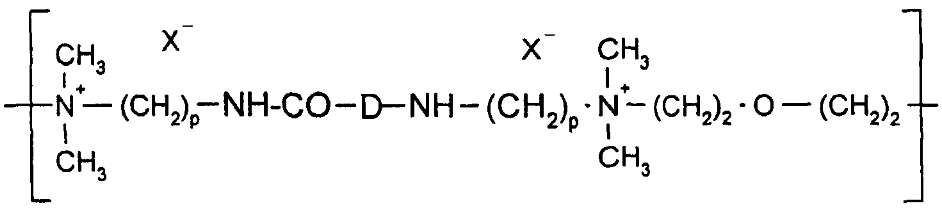

(IX)

dans laquelle p désigne un nombre entier variant de 1 à 6 environ, D peut être nul ou peut représenter un groupement $-(CH_2)_r$ -CO- dans lequel r désigne un nombre égal à 4 ou à 7,
$X^-$ est un anion ;
De tels polymères peuvent être préparés selon les procédés décrits dans les brevets U.S.A. n° 4 157 388, 4 702 906, 4 719 282. Ils sont notamment décrits dans la demande de brevet EP-A-122 324.
Parmi eux, on peut par exemple citer, les produits "Mirapol A 15", "Mirapol AD1", "Mirapol AZ1" et "Mirapol 175" vendus par la société Miranol.

**(12)** Les polymères quaternaires de vinylpyrrolidone et de vinylimidazole tels que par exemple les produits commercialisés sous les dénominations Luviquat FC 905, FC 550 et FC 370 par la société B.A.S.F.

**(13)** Les polyamines comme le Polyquart H vendu par HENKEL, référencé sous le nom de " POLYETHYLENEGLYCOL (15) TALLOW POLYAMINE " dans le dictionnaire CTFA.

**(14)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères constitués de motifs récurrents de formules (W) et (U) suivantes :

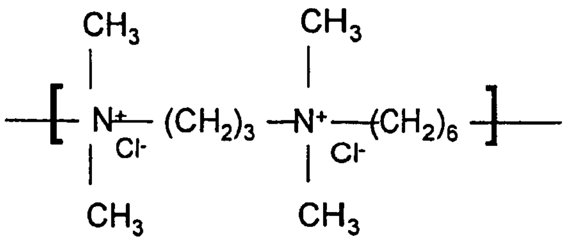

**(W)**

et notamment ceux dont la masse molaire moyenne en poids, déterminée par chromatographie par perméation de gel, est comprise entre 9500 et 9900 ;

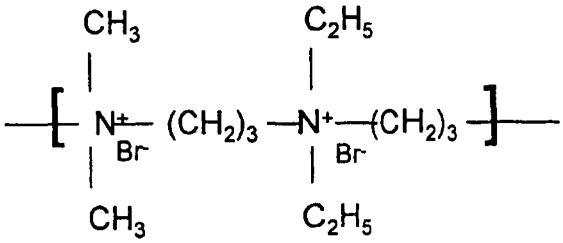

**(U)**

et notamment ceux dont la masse molaire moyenne en poids, déterminée par chromatographie par perméation de gel, est d'environ 1200.

La concentration en polymère(s) cationique(s) autre(s) que les polymères amphiphiles cationiques de la présente invention, dans la composition selon la présente invention, peut varier de 0,01 à 10% en poids par rapport au poids total de la composition, de préférence de 0,05 à 5% et plus préférentiellement encore de 0,1 à 3%.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes
ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL
Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\{CO-R_{19}-CO-Z\} \qquad \textbf{(X)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

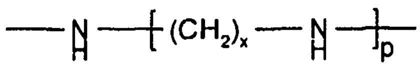

**(XI)**

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

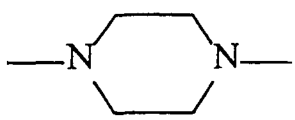

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium

ou de potassium.

(4) Les polymères comportant des motifs zwittérioniques de formule :

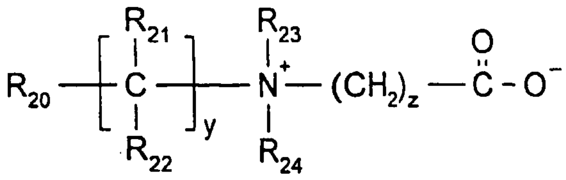

**(XII)**

dans laquelle $R_{20}$ désigne un groupement insaturé polymérisable tel qu'un groupement acylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes:

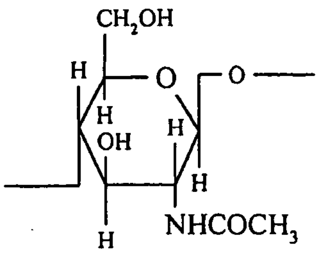

**(XIII)**

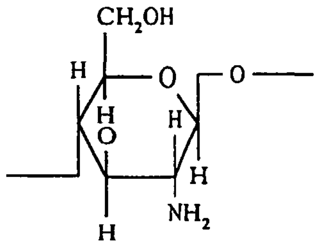

**(XIV)**

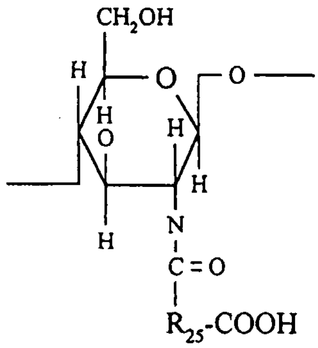

**(XV)**

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule :

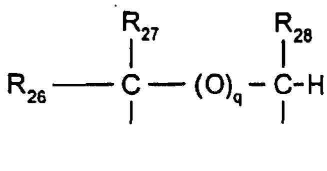

dans laquelle
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XVI) tels que ceux décrits par exemple dans le brevet français 1 400 366 :

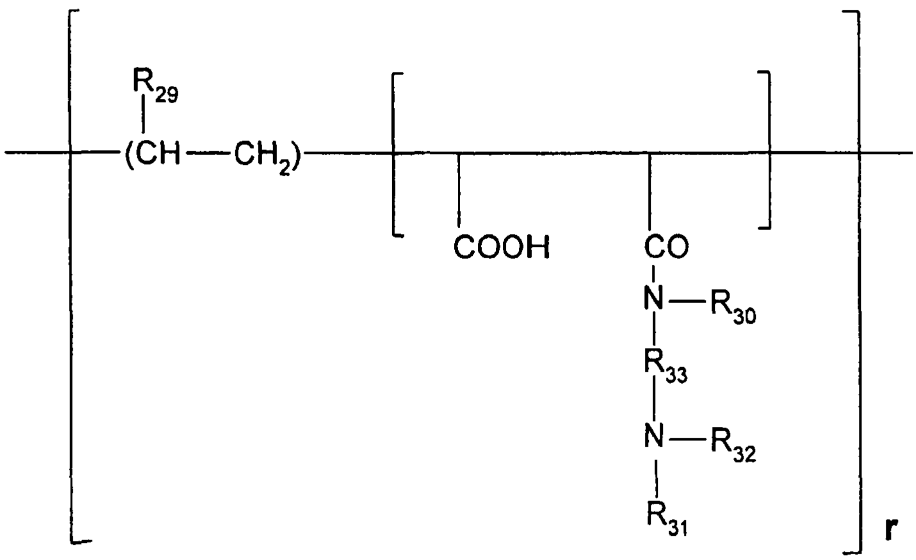

**(XVI)**

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$, $-CH_2-CH_2-CH_2-$, $-CH_2-CH(CH_3)-$, $R_{31}$ ayant les significations mentionnées ci-dessus,
ainsi que les homologues supérieurs de ces radicaux et contenant jusqu'à 6 atomes de carbone.
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- **(XVII)**

où D désigne un radical

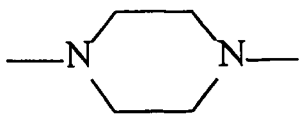

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthane ;

b) les polymères de formule :

-D-X-D-X- **(XVIII)**

où D désigne un radical

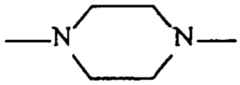

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine.

Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Le milieu de la composition approprié pour la teinture est un milieu aqueux constitué par de l'eau et un ou des solvants hydroxylés décrits ci-dessus.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des polymères non-ioniques ou anioniques, des tensio-actifs cationiques, anioniques ou amphotères, des tensio-actifs non-ioniques autres que ceux de l'invention, des polymères amphiphiles à chaîne grasse autres que ceux de l'invention et en particulier des polymères non-ioniques de type polyuréthanes, des agents épaississants à motifs sucre, des conservateurs, des céramides, des pseudocéramides, des huiles végétales, minérales ou de synthèse, les vitamines ou provitamines comme le panthénol, des opacifiants, etc....

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition selon invention ne soient pas, ou substantiellement pas, altérées par la ou les adjonctions envisagées.

Dans la composition oxydante (B), l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les oxydoréductases à 4 électrons (comme les laccases), les peroxydases et les oxydoréductases à 2 électrons (telles que l'uricase), le cas échéant en présence de leur donneur ou cofacteur respectif.

Le pH de la composition colorante (A) ou de la composition de teinture prête à l'emploi et appliquée sur les fibres kératiniques [composition résultant du mélange de la composition tinctoriale (A) et de la composition oxydante (B)], est généralement compris entre les valeurs 4 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les

alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante :

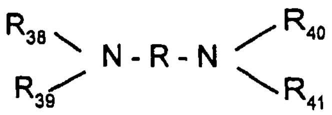

**(XIX)**

dans laquelle R est un reste propylène éventuellement substitué par un groupement hydroxyle ou un radical alkyle en $C_1$-$C_4$ ; $R_{38}$, $R_{39}$, $R_{40}$ et $R_{41}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes environ, et plus préférentiellement de 5 à 45 minutes environ, à rincer les fibres, puis éventuellement à les laver au shampooing, puis à les rincer à nouveau, et à les sécher.

Un exemple concret illustrant l'invention est indiqué ci-après, sans pour autant présenter un caractère limitatif.

**EXEMPLE :**

On a préparé deux compositions de teinture d'oxydation:

- l'une, selon l'invention, composition 1,
  avec un rapport pondéral alcools gras oxyéthylénés /solvant hydroxylé (glycérine) égal à 10,8,
- l'autre, hors invention, composition 2,
  avec un rapport pondéral alcools gras oxyéthylénés /solvant hydroxylé (glycérine) égal à 0,5.

Compositions colorantes :

(exprimées en grammes %)

| **Compositions colorantes** | **1** | **2** |
|---|---|---|
| Alcools gras oxyéthylénés | 32,5 | 5 |
| Glycérine | 3 | 10 |
| Acide oléique | 2 | 2 |
| Alcool oléique | 1,8 | 1,8 |
| Monoisopropanolamide d'acides de coprah | 4 | 4 |
| Polymère amphiphile cationique : Quatrisoft LM 200 vendu par la société AMERCHOL | 0,3 | 0,3 |
| Polymère cationique non amphiphile de formule (W) | 1,8 MA* | 1,8 MA* |
| Polymère amphotère (Merquat 280 de CALGON) | 1,22 MA* | 1,22 MA* |
| Huiles végétales | 0,6 | 0,6 |
| Agent séquestrant, anti-oxydant, réducteur | q.s. | q.s. |
| Ammoniaque (20% d'NH3) | 8 | 8* |
| 1,3-dihydroxybenzène (résorcinol) | 0,011 | 0,011 |

(suite)

| Compositions colorantes | 1 | 2 |
|---|---|---|
| Paraphénylènediamine | 0,31 | 0,31 |
| 1-hydroxy-3-amino-benzène | 0,035 | 0,035 |
| 1-hydroxy-2-amino-benzène | 0,023 | 0,023 |
| 1-hydroxy-4-amino-benzène | 0,53 | 0,53 |
| 5-N-(β-hydroxyéthyl)-amino-2-méthyl-phénol | 1,07 | 1,07 |
| 4-N-méthyl-phénol, sulfate | 0,43 | 0,43 |
| 5-méthyl-2-amino-phénol | 0,12 | 0,12 |
| Eau qsp | 100 | 100 |
| MA* désigne Matière Active | | |

Les compositions colorantes ont été respectivement mélangées, au moment de l'emploi, dans un bol en plastique, à une composition oxydante (décrite ci-dessous), à raison de 1 partie de composition colorante pour 1,5 partie d'oxydant.

| Composition oxydante : | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée | qsp .100 |

On a imprégné des mèches de 3 grammes cheveux naturels à 90% de blancs avec chacun des mélanges ci-dessus obtenus (rapport de bain : 10g de mélange pour 1g de cheveux), et on les a étalées à plat sur un support.
La couleur de ces mèches a ensuite été mesurée au colorimètre MINOLTA CM2002 dans le système L* a* b*, au temps T0, T5 minutes, T10 minutes.
Dans le système L* a* b* les 3 paramètres désignent respectivement l'intensité (L* ), la nuance (a* ), et la saturation (b* ). Selon ce système, plus la valeur de L est élevée, plus la couleur est claire ou peu intense. Inversement, plus la valeur de L est faible, plus la couleur est foncée ou très intense.

Le ΔL par rapport à la valeur de L à T0 (LT0) représente l'évolution de la valeur de L : plus le ΔL est négatif, plus le mélange a foncé rapidement, et donc plus l'oxydation du mélange a été importante.

$$\Delta L5 = LT5 - LT0,$$

$$\Delta L10 = LT10 - LT0.$$

Les résultats ont été réunis dans le tableau (1) suivant :

Tableau (1) : Mesures colorimétriques (moyenne sur 5 mesures)

| | Composition 1 | Composition 2 |
|---|---|---|
| LT0 | 25,7 | 25,8 |
| LT5 | 19,0 | 13,0 |
| **ΔL5** | **-6,7** | **-12,8** |

(suite)

| | Composition 1 | Composition 2 |
|---|---|---|
| LT10 | 14,8 | 7,1 |
| **ΔL10** | **-10,9** | **-18,7** |

Conclusion : Les valeurs de ΔL à 5 et 10 minutes sont beaucoup plus négatives dans le cas de la composition 2 hors invention que dans le cas de la composition 1 selon l'invention, ce qui indique que la couleur du mélange colorant évolue beaucoup moins rapidement dans le cas de l'invention.

## Revendications

1. Composition pour la teinture d'oxydation des fibres kératiniques, en particulier des fibres kératiniques humaines, telles que les cheveux, comprenant, dans un milieu approprié pour la teinture, au moins un colorant d'oxydation, au moins un polymère amphiphile cationique comportant au moins une chaîne grasse, **caractérisée par le fait qu'**elle comprend en outre une association comprenant :

   (I) au moins un alcool gras oxyalkyléné ou glycérolé et,
   (II) au moins un solvant hydroxylé de poids moléculaire inférieur à 250,

   dans une proportion telle que le ratio pondéral (I) / (II) est supérieur à 1.

2. Composition selon la revendication 1, **caractérisée par le fait que** le ou les alcools gras oxyalkylénés sont linéaires ou ramifiés, saturés ou insaturés, et comportent 8 à 40 atomes de carbone et 1 à 250 groupements oxyde d'éthylène et/ou oxyde de propylène.

3. Composition selon la revendication 2, **caractérisée par le fait que** le ou les alcools gras oxyalkylénés sont linéaires ou ramifiés, saturés ou insaturés, et comportent 10 à 20 atomes de carbone et 2 à 40 groupements oxyde d'éthylène.

4. Composition selon la revendication 1, **caractérisée par le fait que** le ou les alcools gras glycérolés sont linéaires ou ramifiés, saturés ou insaturés, et comportent 8 à 40 atomes de carbone et 1 à 30 groupements glycérol.

5. Composition selon l'une quelconque des revendications précédentes, **caractérisée par le fait que** le ou les alcools gras oxyalkylénés ou glycérolés représentent de 0,05 à 50% en poids du poids total de la composition.

6. Composition selon la revendication 5, **caractérisée par le fait que** le ou les alcools gras oxyalkylénés ou glycérolés représentent de 2 à 40% en poids du poids total de la composition.

7. Composition selon la revendication 1, **caractérisée par le fait que** le ou les solvants hydroxylés comportent 2 à 12 atomes de carbone, de préférence 2 à 8 et plus particulièrement 2 à 3 atomes de carbone.

8. Composition selon la revendication 7, **caractérisée par le fait que** le ou les solvants hydroxylés sont choisis parmi l'alcool éthylique, l'alcool propylique, l'alcool n-butylique, le 2-méthyl-1,3-butanediol, le 2,2-diméthyl-1,3-propanediol, le 2-méthyl-1,3-propanediol, le 2-méthyl-2,4-pentanediol, le 1,5-pentanediol, et le 3-méthyl-1,5-pentanediol.

9. Composition selon la revendication 7, **caractérisée par le fait que** le ou les solvants hydroxylés sont choisis parmi la glycérine, le propylène glycol, le dipropylèneglycol et le 1,3-propanediol.

10. Composition selon la revendication 7, **caractérisée par le fait que** le ou les solvants hydroxylés sont choisis parmi les éthers de polyols comportant au moins une fonction hydroxyle libre.

11. Composition selon l'une quelconque des revendications précédentes, **caractérisée par le fait que** le ou les solvants hydroxylés représentent de 0,01 à 25% en poids du poids total de la composition.

12. Composition selon la revendication 11, **caractérisée par le fait que** le ou les solvants hydroxylés représentent de 0,1 à 20% en poids du poids total de la composition.

**13.** Composition selon l'une quelconque des revendications précédentes, **caractérisée par le fait que** le ratio pondéral de l'alcool gras oxyalkyléné ou glycérolé au solvant hydroxylé de poids moléculaire inférieur à 250 est compris entre 1 et 30.

**14.** Composition selon la revendication 13, **caractérisée par le fait que** le ratio pondéral de l'alcool gras oxyalkyléné ou glycérolé au solvant hydroxylé de poids moléculaire inférieur à 250 est compris entre 1,5 et 20.

**15.** Composition selon l'une quelconque des revendications précédentes, **caractérisée par le fait que** le colorant d'oxydation est choisi parmi les bases d'oxydation et/ou les coupleurs.

**16.** Composition selon l'une quelconque des revendications précédentes, **caractérisée par le fait qu'**elle contient au moins une base d'oxydation et au moins un coupleur.

**17.** Composition selon les revendications 15 ou 16, **caractérisée par le fait que** les bases d'oxydation sont choisis parmi les ortho- ou para- phénylènediamines, les bases doubles, les ortho- ou para- aminophénols, et les bases hétérocycliques, ainsi que les sels d'addition de ces composés avec un acide.

**18.** Composition selon la revendication 17, **caractérisée par le fait que** les para-phénylènediamines sont choisies parmi les composés de structure (I) suivante :

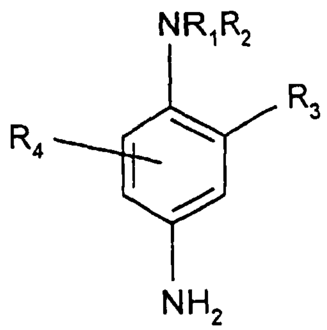

(I)

dans laquelle :

**$R_1$** représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;

**$R_2$** représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

**$R_1$** et **$R_2$** peuvent également former avec l'atome d'azote qui les porte un hétérocycle azoté éventuellement substitué;

**$R_3$** représente un atome d'hydrogène, un atome d'halogène, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,

**$R_4$** représente un atome d'hydrogène, d'halogène ou un radical alkyle en $C_1$-$C_4$.

**19.** Composition selon la revendication 17, **caractérisée par le fait que** les bases doubles sont choisies parmi les composés de structure (II) suivante :

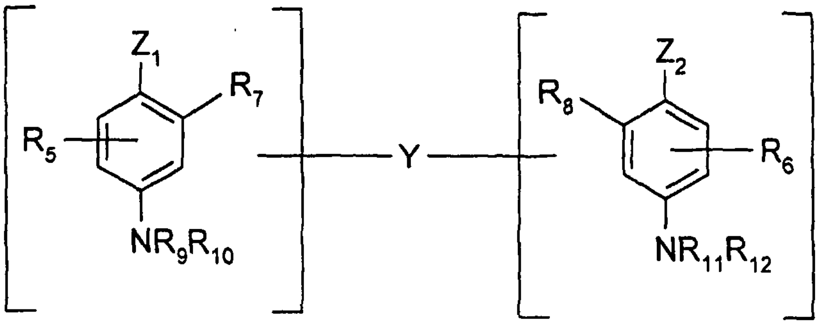

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ; étant entendu que les composés de formule (II) ne comportent qu'un seul bras de liaison Y par molécule.

**20.** Composition selon les revendications 18 ou 19, **caractérisée par le fait que** les groupements azotés sont choisis parmi les radicaux amino, monoalkyl($C_1$-$C_4$)amino, dialkyl($C_1$-$C_4$)amino, trialkyl($C_1$-$C_4$)amino, monohydroxyalkyl ($C_1$-$C_4$)amino, imidazolinium et ammonium.

**21.** Composition selon la revendication 17, **caractérisée par le fait que** les para-aminophénols sont choisis parmi les composés de structure (III) suivante :

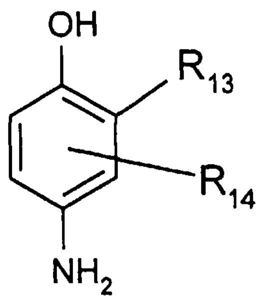

(III)

dans laquelle :

**$R_{13}$** représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

**$R_{14}$** représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

**22.** Composition selon la revendication 17, **caractérisée par le fait que** les bases hétérocycliques sont choisies parmi les dérivés pyridiniques, les dérivés pyrimidiniques, les dérivés pyrazoliques.

**23.** Composition selon les revendications 17 à 22, **caractérisée par le fait que** les bases d'oxydation sont présentes dans des concentrations allant de 0,0005 à 12% en poids par rapport au poids total de la composition.

**24.** Composition selon les revendications 15 ou 16 , **caractérisée par le fait que** les coupleurs sont choisis parmi les métaphénylènediamines, les métaaminophénols, les métadiphénols, les dérivés mono- ou poly-hydroxylés du naphtalène, le sésamol et ses dérivés, les coupleurs hétérocycliques, et les sels d'addition de ces composés avec un acide.

**25.** Composition selon les revendications 15, 16 ou 24, **caractérisée par le fait que** les coupleurs sont présents dans des concentrations allant de 0,0001 à 10% en poids par rapport au poids total de la composition.

**26.** Composition selon l'une quelconque des revendications 15 à 25, **caractérisée par le fait que** les sels d'addition avec un acide des précurseurs de colorants d'oxydation et des coupleurs sont choisis parmi les chlorhydrates, les bromhydrates, les sulfates, les tartrates, les lactates et les acétates.

**27.** Composition selon l'une quelconque des revendications précédentes, **caractérisée par le fait que** le polymère amphiphile cationique comportant au moins une chaîne grasse est choisi parmi :

(i)les celluloses quaternisées modifiées par des groupements comportant au moins une chaîne alkyle, ou arylalkyle, ou alkylaryle comportant au moins 8 atomes de carbone, ou des mélanges de ceux-ci,
(ii)les hydroxyéthylcelluloses quaternisées modifiées par des groupements comportant au moins une chaîne alkyle, ou arylalkyle, ou alkylaryle comportant au moins 8 atomes de carbone, ou des mélanges de ceux-ci,
(iii) les polyuréthanes amphiphiles cationiques avec au moins un groupe hydrophobe comportant de 10 à 30 atomes de carbone,
(iv)les polyvinyllactames amphiphiles cationiques,
(v)le terpolymère acrylique constitué d'acrylates, d'amino(méth)acrylates et d'itaconate d'alkyle en $C_{10}$-$C_{30}$ polyoxyéthyléné à 20 moles d'oxyde d'éthylène.

**28.** Composition selon la revendication 27, **caractérisée par le fait que** les groupes alkyle des celluloses ou des hydroxyéthylcelluloses quaternisées comportent de 8 à 30 atomes de carbone.

**29.** Composition selon les revendications 27 ou 28, **caractérisée par le fait que** le polymère amphiphile cationique est une hydroxyéthylcellulose quaternisée modifiée par un groupement alkyle en $C_{12}$ ou $C_{18}$.

**30.** Composition selon la revendication 27, **caractérisée par le fait que** le polyuréthane amphiphile cationique est un polymère de formule (Ia) suivante :

$$R\text{-}X\text{-}(P)_n\text{-}[L\text{-}(Y)_m]_r\text{-}L'\text{-}(P')_p\text{-}X'\text{-}R' \qquad \text{(Ia)}$$

dans laquelle :

R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ; P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;

la molécule contenant au moins une fonction amine protonée ou quaternisée et au moins un groupement hydrophobe.

**31.** Composition selon l'une quelconque des revendications précédentes, **caractérisée par le fait que** le ou les polymères amphiphiles cationiques comportant au moins une chaîne grasse sont présents en une quantité allant de 0,01 à 3% en poids du poids total de la composition.

**32.** Composition selon la revendication 31, **caractérisée par le fait que** le ou les polymères amphiphiles cationiques comportant au moins une chaîne grasse sont présents en une quantité allant de 0,02 à 0,5% en poids du poids

total de la composition.

**33.** Composition selon l'une quelconque des revendications précédentes, **caractérisée par le fait qu'**elle contient en outre au moins un colorant direct dans la proportion pondérale de 0,001 à 20% en poids par rapport au poids total de la composition.

**34.** Composition selon l'une quelconque des revendications précédentes, **caractérisée par le fait qu'**elle contient en outre au moins un agent réducteur ou anti-oxydant, présent en des quantités allant de 0,05 à 3% en poids par rapport au poids total de la composition.

**35.** Composition selon l'une quelconque des revendications précédentes, **caractérisée par le fait qu'**elle contient en outre un ou plusieurs adjuvants choisis parmi les agents séquestrants, les filtres UV, les cires, les silicones volatiles ou non, cycliques ou linéaires ou ramifiées, organomodifiées (notamment par des groupements amines) ou non, des polymères non-ioniques ou anioniques, des tensio-actifs cationiques, anioniques ou amphotères, des tensio-actifs non-ioniques autres que les alcools gras oxyalkylénés ou polyglycérolés de l'invention, des polymères amphiphiles à chaîne grasse autres que les polymères amphiphiles cationiques à chaîne grasse de l'invention, des agents épaississants à motifs sucre, des conservateurs, des céramides, des pseudocéramides, des huiles végétales, minérales ou de synthèse, des vitamines ou provitamines, des opacifiants.

**36.** Composition prête à l'emploi pour la teinture d'oxydation des fibres kératiniques en particulier des fibres kératiniques humaines telles que les cheveux **caractérisée par le fait qu'**elle est obtenue par mélange d'une composition telle que définie à l'une quelconque des revendications 1 à 35 ou composition (A) et d'une composition (B) contenant au moins un agent oxydant.

**37.** Composition selon la revendication 36, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction, le cas échéant en présence de leur donneur ou cofacteur respectif.

**38.** Composition selon la revendication 37, **caractérisée par le fait que** l'agent oxydant est le peroxyde d'hydrogène.

**39.** Composition selon la revendication 38, **caractérisée par le fait que** l'agent oxydant est une solution d'eau oxygénée dont le titre varie de 1 à 40 volumes.

**40.** Composition selon l'une quelconque des revendications 1 à 39, **caractérisée par le fait qu'**elle possède un pH allant de 4 à 12.

**41.** Composition selon la revendication 36, **caractérisée par le fait que** la composition (A) et/ou la composition (B) contient au moins au moins un polymère cationique différent de ceux de l'invention ou un polymère amphotère.

**42.** Composition selon la revendication 41, **caractérisée par le fait que** le polymère cationique est un poly(ammonium quaternaire) constitué de motifs récurrents répondant à la formule (W) suivante :

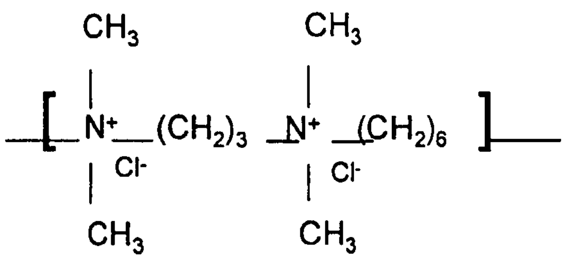

(W)

**43.** Composition selon la revendication 41, **caractérisée par le fait que** le polymère cationique est un poly(ammonium quaternaire) constitué de motifs récurrents répondant à la formule (U) suivante :

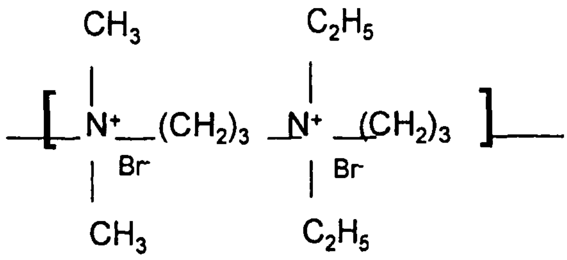 (U)

**44.** Composition selon la revendication 41, **caractérisé par le fait que** le polymère amphotère est un copolymère comprenant au moins comme monomère de l'acide acrylique et un sel de diméthyldiallylammonium.

**45.** Composition selon l'une quelconque des revendications 36 à 44, **caractérisée par le fait que** le ou les polymères cationiques ou amphotères représentent de 0,01% à 10%, de préférence de 0,05% à 5%, et plus préférentiellement de 0,1% à 3% en poids, du poids total de la composition.

**46.** Procédé de teinture des fibres kératiniques et en particulier des fibres kératiniques humaines telles que les cheveux, **caractérisé par le fait qu'**il consiste à appliquer sur les fibres une composition (A) contenant, dans un milieu approprié pour la teinture, au moins un colorant d'oxydation, au moins un polymère amphiphile cationique comportant au moins une chaîne grasse et une association comprenant , au moins un alcool gras oxyalkyléné ou glycérolé (I) et, au moins un solvant hydroxylé de poids moléculaire inférieur à 250 (II), dans une proportion telle que le ratio pondéral (I) / (II) est supérieur à 1, la couleur étant révélée à pH alcalin, neutre ou acide à l'aide d'une composition (B) contenant au moins un agent oxydant qui est mélangée juste au moment de l'emploi à la composition (A) ou qui est appliquée séquentiellement sans rinçage intermédiaire.

**47.** Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins deux compartiments, dont l'un d'entre eux renferme une composition (A) contenant, dans un milieu approprié pour la teinture, au moins un colorant d'oxydation, au moins un polymère amphiphile cationique comportant au moins une chaîne grasse et une association comprenant, au moins un alcool gras oxyalkyléné ou glycérolé (I) et, au moins un solvant hydroxylé de poids moléculaire inférieur à 250 (II), dans une proportion telle que le ratio pondéral (I) / (II) est supérieur à 1, et un autre compartiment renferme une composition (B) comprenant un agent oxydant dans un milieu approprié pour la teinture.

## Claims

**1.** Composition for the oxidation dyeing of keratin fibres, in particular of human keratin fibres such as the hair, comprising, in a medium which is suitable for dyeing, at least one oxidation dye, at least one cationic amphiphilic polymer comprising at least one fatty chain and **characterized in that** it also comprises a combination comprising:

(I) at least one oxyalkylenated or glycerolated fatty alcohol, and
(II) at least one hydroxylated solvent with a molecular weight of less than 250, in a proportion such that the weight ratio (I)/(II) is greater than 1.

**2.** Composition according to Claim 1, **characterized in that** the oxyalkylenated fatty alcohol(s) is (are) linear or branched, and saturated or unsaturated, and comprise 8 to 40 carbon atoms and 1 to 250 ethylene oxide or propylene oxide groups.

**3.** Composition according to Claim 2, **characterized in that** the oxyalkylenated fatty alcohol(s) is (are) linear or branched, and saturated or unsaturated, and comprise 10 to 20 carbon atoms and 2 to 40 ethylene oxide groups.

**4.** Composition according to Claim 1, **characterized in that** the glycerolated fatty alcohol(s) is (are) linear or branched, and saturated or unsaturated, and comprise 8 to 40 carbon atoms and 1 to 30 glycerol groups.

**5.** Composition according to any one of the preceding claims, **characterized in that** the oxyalkylenated or glycerolated

fatty alcohol(s) represent(s) from 0.05% to 50% by weight relative to the total weight of the composition.

6. Composition according to Claim 5, **characterized in that** the oxyalkylenated or glycerolated fatty alcohol(s) represent (s) from 2% to 40% by weight relative to the total weight of the composition.

7. Composition according to Claim 1, **characterized in that** the hydroxylated solvent(s) comprise(s) 2 to 12 carbon atoms, preferably 2 to 8 and more particularly 2 to 3 carbon atoms.

8. Composition according to Claim 7, **characterized in that** the hydroxylated solvent(s) is (are) chosen from ethyl alcohol, propyl alcohol, n-butyl alcohol, 2-methyl-1,3-butanediol, 2,2-dimethyl-1,3-propanediol, 2-methyl-1,3-propanediol, 2-methyl-2,4-pentanediol, 1,5-pentanediol and 3-methyl-1,5-pentanediol.

9. Composition according to Claim 7, **characterized in that** the hydroxylated solvent(s) is (are) chosen from glycerol, propylene glycol, dipropylene glycol and 1,3-propanediol.

10. Composition according to Claim 7, **characterized in that** the hydroxylated solvent(s) is (are) chosen from polyol ethers comprising at least one free hydroxyl function.

11. Composition according to any one of the preceding claims, **characterized in that** the hydroxylated solvent(s) represent(s) from 0.01% to 25% by weight relative to the total weight of the composition.

12. Composition according to Claim 11, **characterized in that** the hydroxylated solvent(s) represent(s) from 0.1% to 20% by weight relative to the total weight of the composition.

13. Composition according to any one of the preceding claims, **characterized in that** the weight ratio of the oxyalkylenated or glycerolated fatty alcohol to the hydroxylated solvent with a molecular weight of less than 250 is between 1 and 30.

14. Composition according to Claim 13, **characterized in that** the weight ratio of the oxyalkylenated or glycerolated fatty alcohol to the hydroxylated solvent with a molecular weight of less than 250 is between 1.5 and 20.

15. Composition according to any one of the preceding claims, **characterized in that** the oxidation dye is chosen from oxidation bases and/or couplers.

16. Composition according to any one of the preceding claims, **characterized in that** it contains at least one oxidation base and at least one coupler.

17. Composition according to Claim 15 or 16, **characterized in that** the oxidation bases are chosen from ortho- or para-phenylenediamines, double bases, ortho- or para-aminophenols and heterocyclic bases, as well as the addition salts of these compounds with an acid.

18. Composition according to Claim 17, **characterized in that** the para-phenylenediamines are chosen from the compounds of structure (I) below:

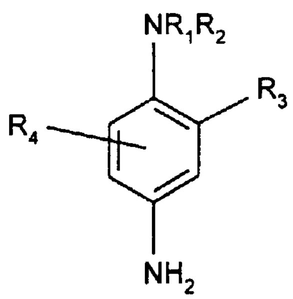

(I)

in which:

**$R_1$** represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical, a $C_1$-$C_4$ alkyl radical substituted with a nitrogenous, phenyl or 4'-aminophenyl group;

**$R_2$** represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical or a $C_1$-$C_4$ alkyl radical substituted with a nitrogenous group;

**$R_1$** and **$R_2$** may also form an optionally substituted, nitrogenous heterocycle with the nitrogen atom which bears them;

**$R_3$** represents a hydrogen atom, a halogen atom, a $C_1$-$C_4$ alkyl radical, a sulfo radical, a carboxy radical, a $C_1$-$C_4$ monohydroxyalkyl radical, a $C_1$-$C_4$ hydroxyalkoxy radical, an acetylamino($C_1$-$C_4$)alkoxy radical, a mesylamino($C_1$-$C_4$)alkoxy radical or a carbamoylamino($C_1$-$C_4$)alkoxy radical,

**$R_4$** represents a hydrogen or halogen atom or a $C_1$-$C_4$ alkyl radical.

**19.** Composition according to Claim 17, **characterized in that** the double bases are chosen from the compounds of structure (II) below:

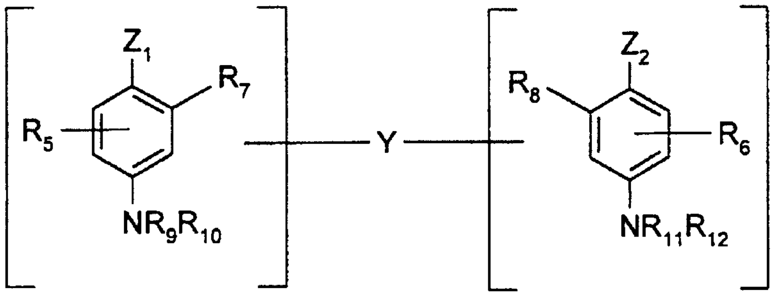

**(II)**

in which:

- $Z_1$ and $Z_2$, which may be identical or different, represent a hydroxyl or $-NH_2$ radical which may be substituted with a $C_1$-$C_4$ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms such as oxygen, sulfur or nitrogen atoms, and optionally substituted with one or more hydroxyl or $C_1$-$C_6$ alkoxy radicals;
- $R_5$ and $R_6$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a linker arm Y;
- $R_7$, $R_8$, $R_9$, $R_{10}$, $R_{11}$ and $R_{12}$, which may be identical or different, represent a hydrogen atom, a linker arm Y or a $C_1$-$C_4$ alkyl radical; it being understood that the compounds of formula (II) contain only one linker arm Y per molecule.

**20.** Composition according to Claim 18 or 19, **characterized in that** the nitrogenous groups are chosen from amino, mono($C_1$-$C_4$)alkylamino, di($C_1$-$C_4$)alkylamino, tri($C_1$-$C_4$)alkylamino, monohydroxy($C_1$-$C_4$)alkylamino, imidazolinium and ammonium radicals.

**21.** Composition according to Claim 17, **characterized in that** the para-aminophenols are chosen from the compounds of structure (III) below:

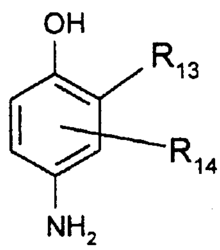

**(III)**

in which:

**$R_{13}$** represents a hydrogen atom, a halogen atom such as fluorine, a $C_1$-$C_4$ alkyl, $C_1$-$C_4$ monohydroxyalkyl, ($C_1$-$C_4$)-alkoxy($C_1$-$C_4$)alkyl, $C_1$-$C_4$ aminoalkyl or hydroxy($C_1$-$C_4$)alkylamino($C_1$-$C_4$)alkyl radical,
**$R_{14}$** represents a hydrogen atom, a halogen atom such as fluorine, a $C_1$-$C_4$-alkyl, $C_1$-$C_4$ monohydroxyalkyl, $C_2$-$C_4$ polyhydroxyalkyl, $C_1$-$C_4$ aminoalkyl, $C_1$-$C_4$ cyanoalkyl or ($C_1$-$C_4$)alkoxy($C_1$-$C_4$)alkyl radical.

**22.** Composition according to Claim 17, **characterized in that** the heterocyclic bases are chosen from pyridine derivatives, pyrimidine derivatives and pyrazole derivatives.

**23.** Composition according to Claims 17 to 22, **characterized in that** the oxidation bases are present in concentrations ranging from 0.0005% to 12% by weight relative to the total weight of the composition.

**24.** Composition according to Claim 15 or 16, **characterized in that** the couplers are chosen from meta-phenylenediamines, meta-aminophenols, meta-diphenols, mono- or polyhydroxylated naphthalene derivatives, sesamol and its derivatives, and heterocyclic couplers, and the addition salts of these compounds with an acid.

**25.** Composition according to Claim 15, 16 or 24, **characterized in that** the couplers are present in concentrations ranging from 0.0001% to 10% by weight relative to the total weight of the composition.

**26.** Composition according to any one of Claims 15 to 25, **characterized in that** the addition salts with an acid of the oxidation dye precursors and couplers are chosen from the hydrochlorides, hydrobromides, sulfates, tartrates, lactates and acetates.

**27.** Composition according to any one of the preceding claims, **characterized in that** the cationic amphiphilic polymer comprising at least one fatty chain is chosen from:

(i) quaternized celluloses modified with groups comprising at least one alkyl, arylalkyl or alkylaryl chain comprising at least 8 carbon atoms, or mixtures thereof,
(ii) quaternized hydroxyethylcelluloses modified with groups comprising at least one alkyl, arylalkyl or alkylaryl chain comprising at least 8 carbon atoms, or mixtures thereof,
(iii) cationic amphiphilic polyurethanes with at least one hydrophobic group comprising from 10 to 30 carbon atoms,
(iv) cationic amphiphilic polyvinyllactams,
(v) the acrylic terpolymer constituted of $C_{10}$-$C_{30}$ alkyl acrylates, amino(meth)acrylates and itaconate, polyoxyethylenated with 20 mol of ethylene oxide.

**28.** Composition according to Claim 27, **characterized in that** the alkyl groups of the quaternized celluloses or hydroxyethylcelluloses comprise from 8 to 30 carbon atoms.

**29.** Composition according to Claim 27 or 28, **characterized in that** the cationic amphiphilic polymer is a quaternized hydroxyethylcellulose modified with a $C_{12}$ or $C_{18}$ alkyl group.

**30.** Composition according to Claim 27, **characterized in that** the cationic amphiphilic polyurethane is a polymer of formula (Ia) below:

$$R\text{-}X\text{-}(P)_n\text{-}[L\text{-}(Y)_m]_r\text{-}L'\text{-}(P')_p\text{-}X'\text{-}R' \qquad \text{(Ia)}$$

in which:

R and R', which may be identical or different, represent a hydrophobic group or a hydrogen atom;
X and X', which may be identical or different, represent a group comprising an amine function optionally comprising a hydrophobic group, or a group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,

n, m and p are each, independently of each other, between 0 and 1000;

the molecule containing at least one protonated or quaternized amine function and at least one hydrophobic group.

**31.** Composition according to any one of the preceding claims, **characterized in that** the cationic amphiphilic polymer (s) comprising at least one fatty chain is (are) present in an amount ranging from 0.01% to 3% by weight relative to the total weight of the composition.

**32.** Composition according to Claim 31, **characterized in that** the cationic amphiphilic polymer(s) comprising at least one fatty chain is (are) present in an amount ranging from 0.02% to 0.5% by weight relative to the total weight of the composition.

**33.** Composition according to any one of the preceding claims, **characterized in that** it also contains at least one direct dye in a weight proportion of from 0.001% to 20% by weight relative to the total weight of the composition.

**34.** Composition according to any one of the preceding claims, **characterized in that** it also contains at least one reducing agent or antioxidant, present in amounts ranging from 0.05% to 3% by weight relative to the total weight of the composition.

**35.** Composition according to any one of the preceding claims, **characterized in that** it also contains one or more adjuvants chosen from sequestering agents, UV screening agents, waxes, volatile or nonvolatile, cyclic or linear or branched silicones, which are optionally organomodified (in particular with amine groups), nonionic or anionic polymers, cationic, anionic or amphoteric surfactants, nonionic surfactants other than the oxyalkylenated or polyglycerolated fatty alcohols of the invention, amphiphilic polymers containing a fatty chain other than the cationic amphiphilic polymers with a fatty chain of the invention, thickeners containing sugar units, preserving agents, ceramides, pseudoceramides, plant, mineral or synthetic oils, vitamins, provitamins, and opacifiers.

**36.** Ready-to-use composition for the oxidation dyeing of keratin fibres, in particular of human keratin fibres such as the hair, **characterized in that** it is obtained by mixing a composition as defined in any one of Claims 1 to 35 or composition (A) and a composition (B) containing at least one oxidizing agent.

**37.** Composition according to Claim 36, **characterized in that** the oxidizing agent is chosen from hydrogen peroxide, urea peroxide, alkali metal bromates or ferricyanides, persalts and redox enzymes, where appropriate in the presence of their respective donor or co-factor.

**38.** Composition according to Claim 37, **characterized in that** the oxidizing agent is hydrogen peroxide.

**39.** Composition according to Claim 38,
**characterized in that** the oxidizing agent is an aqueous hydrogen peroxide solution with a titre ranging from 1 to 40 volumes.

**40.** Composition according to any one of Claims 1 to 39, **characterized in that** it has a pH ranging from 4 to 12.

**41.** Composition according to Claim 36, **characterized in that** composition (A) and/or composition (B) contains at least one cationic polymer other than those of the invention or an amphoteric polymer.

**42.** Composition according to Claim 41, **characterized in that** the cationic polymer is a poly(quaternary ammonium) constituted of repeating units corresponding to formula (W) below:

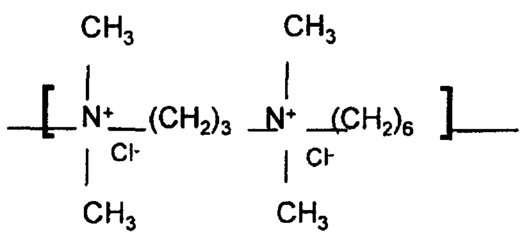

(W)

**43.** Composition according to Claim 41, **characterized in that** the cationic polymer is a poly(quaternary ammonium) constituted of repeating units corresponding to formula (U) below:

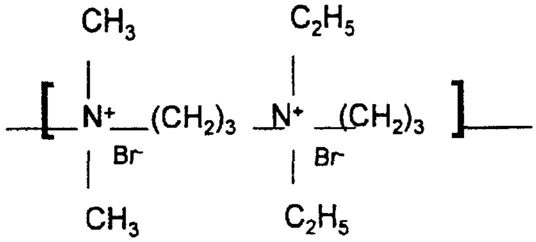

(U)

**44.** Composition according to Claim 41, **characterized in that** the amphoteric polymer is a copolymer comprising at least one acrylic acid monomer and a dimethyldiallylammonium salt.

**45.** Composition according to any one of Claims 36 to 44, **characterized in that** the cationic or amphoteric polymer(s) represent(s) from 0.01% to 10%, preferably from 0.05% to 5% and more preferably from 0.1% to 3% by weight relative to the total weight of the composition.

**46.** Process for dyeing keratin fibres, and in particular human keratin fibres such as the hair, **characterized in that** it consists in applying to the fibres a composition (A) containing, in a medium which is suitable for dyeing, at least one oxidation dye, at least one cationic amphiphilic polymer comprising at least one fatty chain and a combination comprising at least one oxyalkylenated or glycerolated fatty alcohol (I) and at least one hydroxylated solvent with a molecular weight of less than 250 (II), in a proportion such that the weight ratio (I)/(II) is greater than 1, the colour being developed at alkaline, neutral or acidic pH with the aid of a composition (B) containing at least one oxidizing agent which is mixed with composition (A) just at the time of use, or which is applied sequentially without intermediate rinsing.

**47.** Multi-compartment device or "kit" for the oxidation dyeing of keratin fibres, and in particular of human keratin fibres such as the hair, **characterized in that** it comprises at least two compartments, one of which contains a composition (A) containing, in a medium which is suitable for dyeing, at least one oxidation dye, at least one cationic amphiphilic polymer comprising at least one fatty chain and a combination comprising at least one oxyalkylenated or glycerolated fatty alcohol (I) and at least one hydroxylated solvent with a molecular weight of less than 250 (II), in a proportion such that the weight ratio (I)/(II) is greater than 1, and another compartment of which contains a composition (B) comprising an oxidizing agent, in a medium which is suitable for dyeing.

**Patentansprüche**

**1.** Zusammensetzung zum oxidativen Färben von Keratinfasern, insbesondere menschlichen Keratinfasern, wie Haaren, die in einem zum Färben geeigneten Medium mindestens einen Oxidationsfarbstoff und mindestens ein kationisches amphiphiles Polymer enthält, das mindestens eine Fettkette aufweist, **dadurch gekennzeichnet, dass** sie ferner eine Kombination enthält, umfassend:

(I) mindestens einen alkoxylierten oder mit Glycerin veretherten Fettalkohol, und
(II) mindestens ein hydroxyliertes Lösungsmittel mit einer Molmasse unter 250

in einem solchen Mengenanteil, dass das Gewichtsverhältnis (I)/(II) über 1 liegt.

**2.** Zusammensetzung nach Anspruch 1, **dadurch gekennzeichnet, dass** der oder die alkoxylierten Fettalkohole linear oder verzweigt, gesättigt oder ungesättigt vorliegen und 8 bis 40 Kohlenstoffatome und 1 bis 250 Ethylenoxidgruppen und/oder Propylenoxidgruppen aufweisen.

**3.** Zusammensetzung nach Anspruch 2, **dadurch gekennzeichnet, dass** der oder die alkoxylierten Fettalkohole geradkettig oder verzweigt, gesättigt oder ungesättigt vorliegen und 10 bis 20 Kohlenstoffatome aufweisen und 2 bis 40 Ethylenoxidgruppen besitzen.

**4.** Zusammensetzung nach Anspruch 1, **dadurch gekennzeichnet, dass** der oder die mit Glycerin veretherten Alkohole geradkettig oder verzweigt, gesättigt oder ungesättigt vorliegen und 8 bis 40 Kohlenstoffatome und 1 bis 30 Glyceringruppen enthalten.

**5.** Zusammensetzung nach einem der vorhergehenden Ansprüche, **dadurch gekennzeichnet, dass** der oder die alkoxylierten oder mit Glycerin veretherten Fettalkohole 0,05 bis 50 Gew.-% des Gesamtgewichts der Zusammensetzung ausmachen.

**6.** Zusammensetzung nach Anspruch 5, **dadurch gekennzeichnet, dass** der oder die alkoxylierten oder mit Glycerin veretherten Fettalkohole 2 bis 40 Gew.-% des Gesamtgewichts der Zusammensetzung ausmachen.

**7.** Zusammensetzung nach Anspruch 1, **dadurch gekennzeichnet, dass** das oder die hydroxylierten Lösungsmittel 2 bis 12 Kohlenstoffatome, vorzugsweise 2 bis 8 Kohlenstoffatome und insbesondere 2 bis 3 Kohlenstoffatome aufweisen.

**8.** Zusammensetzung nach Anspruch 7, **dadurch gekennzeichnet,**
**dass** das oder die hydroxylierten Lösungsmittel unter Ethanol, Propylalkohol, n-Butylalkohol, 2-Methyl-1,3-butandiol, 2,2-Dimethyl-1,3-propandiol, 2-Methyl-1,3-propandiol, 2-Methyl-2,4-pentandiol, 1,5-Pentandiol und 3-Methyl-1,5-pentandiol ausgewählt sind.

**9.** Zusammensetzung nach Anspruch 7, **dadurch gekennzeichnet, dass** das oder die hydroxylierten Lösungsmittel unter Glycerin, Propylenglycol, Dipropylenglycol und 1,3-Propandiol ausgewählt sind.

**10.** Zusammensetzung nach Anspruch 7, **dadurch gekennzeichnet, dass** das oder die hydroxylierten Lösungsmittel unter den Polyolethern ausgewählt sind, die mindestens eine freie Hydroxyfunktion besitzen.

**11.** Zusammensetzung nach einem der vorhergehenden Ansprüche, **dadurch gekennzeichnet, dass** das oder die hydroxylierten Lösungsmittel 0,01 bis 25 Gew.-% des Gesamtgewichts der Zusammensetzung ausmachen.

**12.** Zusammensetzung nach Anspruch 11, **dadurch gekennzeichnet, dass** das oder die hydroxylierten Lösungsmittel 0,1 bis 20 Gew.-% des Gesamtgewichts der Zusammensetzung ausmachen.

**13.** Zusammensetzung nach einem der vorhergehenden Ansprüche, **dadurch gekennzeichnet, dass** das Gewichtsverhältnis des alkoxylierten oder mit Glycerin veretherten Fettalkohols und des hydroxylierten Lösungsmittels mit einer Molmasse unter 250 im Bereich von 1 bis 30 liegt.

**14.** Zusammensetzung nach Anspruch 13, **dadurch gekennzeichnet, dass** das Gewichtsverhältnis des alkoxylierten oder mit Glycerin veretherten Fettalkohols und des hydroxylierten Lösungsmittels mit einer Molmasse unter 250 im Bereich von 1,5 bis 20 liegt.

**15.** Zusammensetzung nach einem der vorhergehenden Ansprüche, **dadurch gekennzeichnet, dass** das Oxidationsmittel unter den Oxidationsbasen und/oder Kupplern ausgewählt ist.

**16.** Zusammensetzung nach einem der vorhergehenden Ansprüche, **dadurch gekennzeichnet, dass** sie mindestens eine Oxidationsbase und mindestens einen Kuppler enthält.

**17.** Zusammensetzung nach den Ansprüchen 15 oder 16, **dadurch gekennzeichnet, dass** die Oxidationsbasen unter den *ortho*- oder *para*-Phenylendiaminen, Doppelbasen, *ortho*- oder *para*-Aminophenolen und heterocyclischen Basen sowie den Additionssalzen dieser Verbindungen mit einer Säure ausgewählt sind.

**18.** Zusammensetzung nach Anspruch 17, **dadurch gekennzeichnet, dass** die *para*-Phenylendiamine unter den Verbindungen der folgenden Struktur (I) ausgewählt sind:

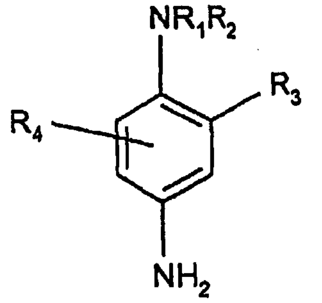

(I)

wobei in der Formel:

$R_1$ bedeutet ein Wasserstoffatom, eine Alkyl($C_{1-4}$)gruppe, eine Monohydroxyalkyl($C_{1-4}$)gruppe, eine Polyhydroxyalkyl($C_{2-4}$)gruppe, eine Alkoxy($C_{1-4}$)alkyl($C_{1-4}$)gruppe, eine Alkyl($C_{1-4}$)gruppe, die mit einer stickstoffhaltigen Gruppe substituiert ist, Phenyl oder 4'-Aminophenyl;
$R_2$ bedeutet ein Wasserstoffatom, eine Alkyl($C_{1-4}$)gruppe, eine Monohydroxyalkyl($C_{1-4}$)gruppe oder eine Polyhydroxyalkyl($C_{2-4}$)-gruppe, eine Alkoxy($C_{1-4}$)alkyl($C_{1-4}$)gruppe oder eine Alkyl($C_{1-4}$)-gruppe, die mit einer stickstoffhaltigen Gruppe substituiert ist; $R_1$ und $R_2$ können auch mit dem Stickstoffatom, das sie trägt, einen gegebenenfalls substituierten Stickstoffheterocyclus bilden;
$R_3$ bedeutet ein Wasserstoffatom, ein Halogenatom, eine Alkyl($C_{1-4}$)gruppe, Sulfo, Carboxy, eine Monohydroxyalkyl($C_{1-4}$)-gruppe, eine Hydroxyalkoxy($C_{1-4}$)gruppe, eine Acetylaminoalk-oxy($C_{1-4}$)gruppe, eine Mesylaminoalkoxy($C_{1-4}$)gruppe oder eine Carbamoylaminoalkoxy($C_{1-4}$)gruppe;
$R_4$ bedeutet ein Wasserstoffatom, ein Halogenatom oder eine Alkyl($C_{1-4}$)gruppe.

**19.** Zusammensetzung nach Anspruch 17, **dadurch gekennzeichnet, dass** die Doppelbasen unter den Verbindungen der folgenden Struktur (II) ausgewählt sind:

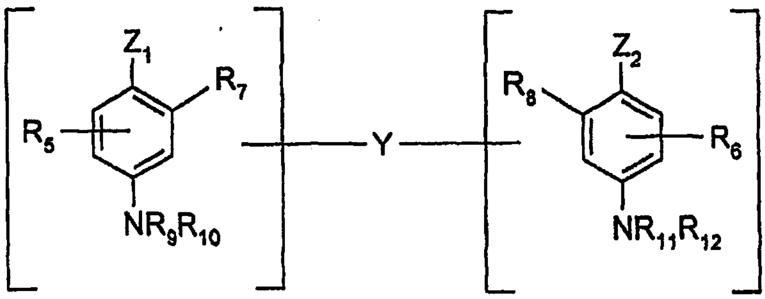

(II)

wobei in der Formel:

- $Z_1$ und $Z_2$, die gleich oder verschieden sind, eine Hydroxygruppe oder eine Gruppe $-NH_2$ bedeuten, die mit einer Alkyl($C_{1-4}$)gruppe oder einer Verbindungsgruppe Y substituiert sein können;
- die Verbindungsgruppe Y bedeutet ein geradkettige oder verzweigte Alkylenkette mit 1 bis 14 Kohlenstoffatomen, die durch eine oder mehrere stickstoffhaltige Gruppen und/oder durch ein oder mehrere Heteroatome, wie Sauerstoff, Schwefel oder Stickstoff, unterbrochen oder abgeschlossen sein kann und gegebenenfalls mit einer oder mehreren Hydroxygruppen oder Alkoxy($C_{1-6}$)gruppen substituiert ist;
- $R_5$ und $R_6$ bedeuten ein Wasserstoffatom oder ein Halogenatom, eine Alkyl($C_{1-4}$)gruppe, eine Monohydroxyalkyl($C_{1-4}$)-gruppe, eine Polyhydroxyalkyl($C_{2-4}$)gruppe, eine Aminoalkyl-($C_{1-4}$)gruppe oder eine Verbindungsgruppe Y;
- $R_7$, $R_8$, $R_9$, $R_{10}$, $R_{11}$ und $R_{12}$, die gleich oder verschieden sind, bedeuten ein Wasserstoffatom, eine Verbindungsgruppe Y oder eine Alkyl($C_{1-4}$)gruppe;
mit der Maßgabe, dass die Verbindungen der Formel (II) nur eine Verbindungsgruppe Y pro Molekül aufweisen.

**20.** Zusammensetzung nach den Ansprüchen 18 oder 19, **dadurch gekennzeichnet, dass** die stickstoffhaltigen Gruppen unter den Gruppen Amino, Monalkyl($C_{1-4}$)amino, Dialkyl($C_{1-4}$)amino, Trialkyl($C_{1-4}$)amino, Monohydroxyalkyl ($C_{1-4}$)amino, Imidazolinium und Ammonium ausgewählt sind.

**21.** Zusammensetzung nach Anspruch 17, **dadurch gekennzeichnet, dass** die *para*-Aminophenole unter den Verbindungen der folgenden Struktur (III) ausgewählt sind:

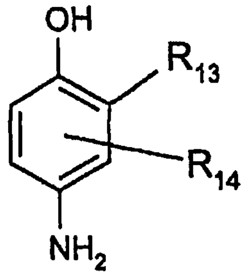

**(III)**

wobei in der Formel:

$R_{13}$ bedeutet ein Wasserstoffatom, ein Halogenatom, wie Fluor, eine Alkyl($C_{1-4}$)gruppe, eine Monohydroxyalkyl ($C_{1-4}$)gruppe, eine Alkoxy($C_{1-4}$)alkyl($C_{1-4}$)gruppe oder eine Aminoalkyl($C_{1-4}$)gruppe oder eine Hydroxyalkyl ($C_{1-4}$)aminoalkyl($C_{1-4}$)gruppe,
$R_{14}$ ein Wasserstoffatom oder ein Halogenatom, wie Fluor, eine Alkyl($C_{1-4}$)gruppe, eine Monohydroxyalkyl($C_{1-4}$) gruppe, eine Polyhydroxyalkyl($C_{2-4}$)gruppe, eine Aminoalkyl($C_{1-4}$)gruppe, eine Cyanoalkyl($C_{1-4}$)gruppe oder eine Alkoxy($C_{1-4}$)alkyl($C_{1-4}$)gruppe.

**22.** Zusammensetzung nach Anspruch 17, **dadurch gekennzeichnet, dass** die heterocyclischen Basen unter den Pyridinderivaten, Pyrimidinderivaten, Pyrazolderivaten ausgewählt sind.

**23.** Zusammensetzung nach den Ansprüchen 17 bis 22, **dadurch gekennzeichnet, dass** die Oxidationsbasen in Konzentrationen von 0,0005 bis 12 Gew.-%, bezogen auf das Gesamtgewicht der Zusammensetzung, enthalten sind.

**24.** Zusammensetzung nach den Ansprüchen 15 oder 16, **dadurch gekennzeichnet, dass** die Kuppler unter den *m*-Phenylendiaminen, *m*-Aminophenolen, *m*-Dihydroxybenzolen, Mono- oder Polyhydroxyderivaten von Naphthalin, Sesamol und seinen Derivaten, heterocyclischen Kupplern und den Additionssalzen dieser Verbindungen mit einer Säure ausgewählt sind.

**25.** Zusammensetzung nach den Ansprüchen 15, 16 oder 24, **dadurch gekennzeichnet, dass** die Kuppler in Konzentrationen von 0,0001 bis 10 Gew.-%, bezogen auf das Gesamtgewicht der Zusammensetzung, enthalten sind.

**26.** Zusammensetzung nach einem der Ansprüche 15 bis 25, **dadurch gekennzeichnet, dass** die Additionssalze der Farbstoffvorprodukte von Oxidationsfarbstoffen und Kuppler mit einer Säure unter den Hydrochloriden, Hydrobromiden, Sulfaten, Tartraten, Lactaten und Acetaten ausgewählt sind.

**27.** Zusammensetzung nach einem der vorhergehenden Ansprüche, **dadurch gekennzeichnet, dass** das kationische amphiphile Polymer, das mindestens eine Fettkette enthält, ausgewählt ist unter:

(i) quaternisierten Cellulosen, die mit Gruppen modifiziert sind, die mindestens eine Alkylkette oder Arylalkylkette oder Alkylarylkette mit mindestens 8 Kohlenstoffatomen aufweisen, oder deren Gemischen modifiziert sind,
(ii) quaternisierten Hydroxyethylcellulosen, die mit Gruppen, die mindestens eine Alkylkette oder Arylalkylkette oder Alkylarylkette mit mindestens 8 Kohlenstoffatomen aufweisen, oder deren Gemischen modifiziert sind,
(iii) kationischen amphiphilen Polyurethanen mit mindestens einer hydrophoben Gruppe, die 10 bis 30 Kohlenstoffatome aufweist,
(iv) kationischen amphiphilen Polyvinyllactamen,
(v) dem Acrylterpolymer, das aus Acrylaten, Amino(meth)acrylaten und Alkyl($C_{10-30}$)itaconat, das mit 20 mol

Ethylenoxid polyethoxyliert ist, besteht.

**28.** Zusammensetzung nach Anspruch 27, **dadurch gekennzeichnet, dass** die Alkylgruppen der quaternisierten Cellulosen oder Hydroxycellulosen 8 bis 30 Kohlenstoffatome aufweisen.

**29.** Zusammensetzung nach den Ansprüchen 27 oder 28, **dadurch gekennzeichnet, dass** das kationische amphiphile Polymer eine quaternisierte Hydroxyethylcellulose ist, die mit einer $C_{12}$- oder $C_{18}$-Alkylgruppe modifiziert ist.

**30.** Zusammensetzung nach Anspruch 27, **dadurch gekennzeichnet, dass** das kationische amphiphile Polyurethan ein Polymer der folgenden Formel (I) ist:

$$\mathbf{R\text{-}X\text{-}(P)_n\text{-}[L\text{-}(Y)_m]_r\text{-}L'\text{-}(P')_p\text{-}X'\text{-}R'} \qquad \text{(Ia)}$$

worin bedeuten:

R und R', die gleich oder verschieden sind, eine hydrophobe Gruppe oder ein Wasserstoffatom;
X und X', die gleich oder verschieden sind, eine Gruppe, die eine Aminofunktion aufweist, die gegebenenfalls eine hydrophobe Gruppe trägt, oder auch die Gruppe L";
L, L' und L", die gleich oder verschieden sind, eine Gruppe, die von einem Diisocyanat abgeleitet ist;
P und P', die gleich oder verschieden sind, eine Gruppe, die eine Aminofunktion aufweist, die gegebenenfalls eine hydrophobe Gruppe trägt;
Y eine hydrophile Gruppe;
r eine ganze Zahl von 1 bis 100, vorzugsweise 1 bis 50 und insbesondere 1 bis 25;
n, m und p Werte, die unabhängig voneinander im Bereich von 0 bis 1 000 liegen;
wobei das Molekül mindestens eine protonierte oder quaternisierte Aminofunktion und mindestens eine hydrophobe Gruppe aufweist.

**31.** Zusammensetzung nach einem der vorhergehenden Ansprüche, **dadurch gekennzeichnet, dass** das oder die kationischen amphiphilen Polymere, die mindestens eine Fettkette aufweisen, in einem Mengenanteil von 0,01 bis 3 Gew.-% des Gesamtgewichts der Zusammensetzung vorliegen.

**32.** Zusammensetzung nach Anspruch 31, **dadurch gekennzeichnet, dass** das oder die kationischen amphiphilen Polymere, die mindestens eine Fettkette aufweisen, in einer Menge von 0,02 bis 0,5 Gew.-% des Gesamtgewichts der Zusammensetzung enthalten sind.

**33.** Zusammensetzung nach einem der vorhergehenden Ansprüche, **dadurch gekennzeichnet, dass** sie ferner mindestens einen Direktfarbstoff in einem Gewichtsverhältnis von 0,001 bis 20 Gew.-%, bezogen auf das Gesamtgewicht der Zusammensetzung, aufweist.

**34.** Zusammensetzung nach einem der vorhergehenden Ansprüche, **dadurch gekennzeichnet, dass** sie außerdem mindestens ein Reduktionsmittel oder Antioxidationsmittel enthält, das in Mengenanteilen von 0,05 bis 3 Gew.-%, bezogen auf das Gesamtgewicht der Zusammensetzung, enthalten ist.

**35.** Zusammensetzung nach einem der vorhergehenden Ansprüche,
**dadurch gekennzeichnet, dass** sie einen oder mehrere Zusatzstoffe enthält, die unter den Maskierungsmitteln, UV-Filtern, Wachsen, flüchtigen oder nichtflüchtigen, cyclischen oder linearen oder verzweigten, organomodifizierten (insbesondere mit Aminogruppen) oder nicht organomodifizierten Siliconen, nichtionischen oder anionischen Polymeren, kationischen, anionischen oder amphoteren grenzflächenaktiven Stoffen, nichtionischen grenzflächenaktiven Stoffen, die von den alkoxylierten oder mehrfach mit Glycerin veretherten Fettalkoholen der Erfindung verschieden sind, amphiphilen Polymeren mit Fettkette, die von den kationischen amphiphilen Polymeren mit Fettkette gemäß der Erfindung verschieden sind, Verdickungsmitteln mit Zuckereinheiten, Konservierungsmitteln, Ceramiden, Pseudoceramiden, pflanzlichen Ölen, Mineralölen oder synthetischen Ölen, Vitaminen oder Provitaminen und Trübungsmitteln ausgewählt sind.

**36.** Gebrauchsfertige Zusammensetzung zum oxidativen Färben von Keratinfasern und insbesondere menschlichen Keratinfasern, wie Haaren, **dadurch gekennzeichnet, dass** sie durch Mischen einer Zusammensetzung, wie sie in einem der Ansprüche 1 bis 35 definiert ist, oder Zusammensetzung (A), und einer Zusammensetzung (B) gebildet wird, die mindestens ein Oxidationsmittel enthält.

**37.** Zusammensetzung nach Anspruch 36, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls in Gegenwart ihres jeweiligen Donors oder Cofaktors ausgewählt ist.

**38.** Zusammensetzung nach Anspruch 37, **dadurch gekennzeichnet, dass** es sich bei dem Oxidationsmittel um Wasserstoffperoxid handelt.

**39.** Zusammensetzung nach Anspruch 38, **dadurch gekennzeichnet, dass** das Oxidationsmittel eine wässrige Wasserstoffperoxidlösung ist, deren Titer im Bereich von 1 bis 40 Volumina liegt.

**40.** Zusammensetzung nach einem der Ansprüche 1 bis 39, **dadurch gekennzeichnet, dass** sie einen pH-Wert von 4 bis 12 aufweist.

**41.** Zusammensetzung nach Anspruch 36, **dadurch gekennzeichnet, dass** die Zusammensetzung (A) und/oder die Zusammensetzung (B) mindestens ein kationisches Polymer enthalten, das von den erfindungsgemäßen Polymeren verschieden ist, oder ein amphoteres Polymer enthalten.

**42.** Zusammensetzung nach Anspruch 41, **dadurch gekennzeichnet, dass** das kationische Polymer ein polyquartäres Ammonium ist, das aus Wiederholungseinheiten der folgenden Formel (W) besteht:

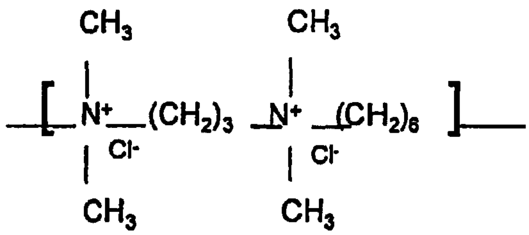

(W)

**43.** Zusammensetzung nach Anspruch 41, **dadurch gekennzeichnet, dass** das kationische Polymer ein polyquartäres Ammonium ist, das aus Wiederholungseinheiten der folgenden Formel (U) besteht:

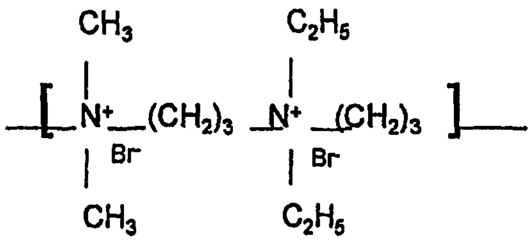

(U)

**44.** Zusammensetzung nach Anspruch 41, **dadurch gekennzeichnet, dass** das amphotere Polymer ein Copolymer ist, das als Monomer zumindest die Acrylsäure und ein Dimethyldiallylammoniumsalz enthält.

**45.** Zusammensetzung nach einem der Ansprüche 36 bis 44, **dadurch gekennzeichnet, dass** das oder die kationischen oder amphoteren Polymere 0,01 bis 10 %, vorzugsweise 0,05 bis 5 % und noch bevorzugter 0,1 bis 3 Gew.-% des Gesamtgewichts der Zusammensetzung ausmachen.

**46.** Verfahren zum Färben von Keratinfasern und insbesondere menschlichen Keratinfasern, wie Haaren, **dadurch gekennzeichnet, dass** es darin besteht, auf die Fasern eine Zusammensetzung (A) aufzutragen, die in einem zum Färben geeigneten Medium mindestens einen Oxidationsfarbstoff, mindestens ein kationisches amphiphiles Polymer, das mindestens eine Fettkette aufweist, und eine Kombination enthält, die mindestens einen alkoxylierten oder mit Glycerin veretherten Fettalkohol (I) und mindestens ein hydroxyliertes Lösungsmittel mit einer Molmasse unter 250 (II) in einem solchen Mengenanteil umfasst, dass das Gewichtsverhältnis (I)/(II) über 1 liegt, wobei die Farbe bei einem alkalischen, neutralen oder sauren pH-Wert mit Hilfe einer Zusammensetzung (B) gebildet wird, die

mindestens ein Oxidationsmittel enthält und die bei der Anwendung mit der Zusammensetzung (A) vermischt wird oder die getrennt davon ohne zwischenzeitliches Spülen anschließend aufgetragen wird.

**47.** Vorrichtung mit mehreren Abteilungen oder "Kit" zum oxidativen Färben von Keratinfasern und insbesondere menschlichen Keratinfasern, wie Haaren, **dadurch gekennzeichnet, dass** sie mindestens zwei Abteilungen aufweist, wobei eine Abteilung eine Zusammensetzung (A) enthält, die in einem zum Färben geeigneten Medium mindestens einen Oxidationsfarbstoff, mindestens ein kationisches amphiphiles Polymer, das mindestens eine Fettkette aufweist, und eine Kombination enthält, die mindestens einen alkoxylierten oder mit Glycerin veretherten Fettalkohol (I) und mindestens ein hydroxyliertes Lösungsmittel mit einer Molmasse unter 250 (II) in einem solchen Mengenanteil umfasst, das das Gewichtsverhältnis (I)/(II) über 1 liegt, und eine andere Abteilung eine Zusammensetzung (B) enthält, die in einem zum Färben geeigneten Medium ein Oxidationsmittel enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0943320 A
- FR 0009609
- WO 0068282 A
- EP 1090623 A
- EP 0824914 A
- EP 0825200 A
- GB 1026978 A
- GB 1153196 A
- DE 2359399
- JP 63169571 A
- JP 3010659 A
- WO 9615765 A
- FR 2750048 A
- DE 3843892
- DE 4133957
- WO 9408969 A
- WO 9408970 A
- FR 2733749 A
- DE 19543988
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- FR 2080759
- FR 2190406
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A
- FR 1400366


### Littérature non-brevet citée dans la description


- CTFA. 1997
- POLYETHYLENEGLYCOL (15) TALLOW POLYAMINE. CTFA